# EUROPEAN PATENT SPECIFICATION

(11) **EP 3 066 218 B1**
(45) Date of publication and mention of the grant of the patent: **26.12.2018**
(21) Application number: 14859794.1
(22) Date of filing: 05.11.2014
(51) Int. Cl.: C12Q 1/68

(54) **METHODS FOR DETECTING NUCLEIC ACIDS**
NACHWEISVERFAHREN FÜR NUKLEINSÄUREN
PROCÉDÉS DE DÉTECTION D'ACIDES NUCLÉIQUES

(30) Priority: 05.11.2013 US 201361900225 P
(43) Date of publication of application: 14.09.2016
(73) Proprietor: HTG Molecular Diagnostics, Inc., Tuscon, AZ 85706 (US)
(72) Inventor: ROUNSEVILLE, Matt, Tucson, AZ 85706 (US); MODUR, Vijay, Tucson, AZ 85706 (US)
(74) Representative: Docherty, Robert Charles
(86) International application number: PCT/US2014/064157
(87) International publication number: WO 2015/069787

(56) References cited:
- WO-A1-2009/101193
- WO-A1-2013/087630
- WO-A1-2014/005038
- WO-A2-00/37683
- WO-A2-2005/001113
- WO-A2-2008/052774
- US-A1- 2012 028 826

### SEQUENCE LISTING

<110> HTG Molecular Disgnostics, Inc. Rounseville, Matt Modur, Vijay
<120> METHODS FOR DETECTING NUCLEIC ACIDS
<130> 8736-91247-03
<150> US 61/900,225
   <151> 2013-11-05
<160> 11
<170> PatentIn version 3.5
<210> 1
   <211> 37
   <212> DNA
   <213> Artificial Sequence
<220>
   <223> Synthetic oligonucleotide - BRAF V600wt
<400> 1
   ggtgattttg gtctagctac agtgaaatct cgatgga 37
<210> 2
   <211> 37
   <212> DNA
   <213> Artificial Sequence
<220>
   <223> Synthetic oligonucleotide - BRAF V600E
<400> 2
   ggtgattttg gtctagctac agagaaatct cgatgga 37
<210> 3
   <211> 37
   <212> DNA
   <213> Artificial Sequence
<220>
   <223> Synthetic oligonucleotide - BRAF V600K
<400> 3
   ggtgattttg gtctagctac aaagaaatct cgatgga 37
<210> 4
   <211> 37
   <212> DNA
   <213> Artificial Sequence
<220>
   <223> Synthetic oligonucleotide - BRAF V600E2
<400> 4
   ggtgattttg gtctagctac agaaaaatct cgatgga 37
<210> 5
   <211> 37
   <212> DNA
   <213> Artificial Sequence
<220>
   <223> Synthetic oligonucleotide - BRAF V600D
<400> 5
   ggtgattttg gtctagctac agataaatct cgatgga 37
<210> 6
   <211> 37
   <212> DNA
   <213> Artificial Sequence
<220>
   <223> Synthetic oligonucleotide - BRAF V600 probe
<400> 6
   tgtagctaga ccaaaatcac ctatttttac tgtgagg 37
<210> 7
   <211> 29
   <212> DNA
   <213> Artificial Sequence
<220>
   <223> Synthetic oligonucleotide - BRAF V600 wt bifunctional target-specific portion
<400> 7
   ctgatgggac ccactccatc gagatttca 29
<210> 8
   <211> 29
   <212> DNA
   <213> Artificial Sequence
<220>
   <223> Synthetic oligonucleotide - BRAF V600E bifunctional target-specific portion
<400> 8
   ctgatgggac ccactccatc gagatttct 29
<210> 9
   <211> 30
   <212> DNA
   <213> Artificial Sequence
<220>
   <223> Synthetic oligonucleotide - BRAF V600K bifunctional target-specific portion
<400> 9
   ctgatgggac ccactccatc gagatttctt 30
<210> 10
   <211> 30
   <212> DNA
   <213> Artificial Sequence
<220>
   <223> Synthetic oligonucleotide - BRAF V600E2 bifunctional target-specific portion
<400> 10
   ctgatgggac ccactccatc gagatttttc 30
<210> 11
   <211> 30
   <212> DNA
   <213> Artificial Sequence
<220>
   <223> Synthetic oligonucleotide - BRAF V600D bifunctional target-specific portion
<400> 11
   ctgatgggac ccactccatc gagatttatc 30

## Description

### FIELD

This disclosure relates to methods of detecting nucleic acid molecules in a sample, including detecting nucleic acid molecules having one or more nucleotide variants.

### BACKGROUND

Many diseases or disorders are characterized by alterations in gene expression and/or genetic changes (for example, mutations) that affect the expression or function of the gene or an encoded protein. Diagnosis, prognosis, and/or selection of therapy for many disorders include determining gene expression levels or identifying particular genetic changes in a sample from a subject known or suspected to have the disorder. There is a continuing need for development of sensitive, specific, and rapid assays for detecting mutations or gene signatures associated with disease.

Methods for detecting nucleic acid sequence variations are known and disclosed in WO2005001113 and WO2008052774.

WO2005001113 discloses methods for detecting target nucleic acid sequence variation in a sample by providing a plurality of oligonucleotide probes comprising each a specific portion representing the polymorphism and a probe complementary to the target comprising a barcode. If both probes are able to hybridize to the target they are ligated and form a ligated probe which can then be detected via the barcode.

WO2008052774 discloses a detection method of a single- or double-stranded nucleic acid molecules by using a bifunctional probe which is anchored via a linker to a solid support. The detection probe binds indirectly to the target nucleic acid via another nucleic acid molecule which is partial complementary to the target nucleic acid.

### SUMMARY

Disclosed herein are methods for detecting the presence and/or amount of a target nucleic acid molecule in a sample, for example to determine whether or not a target nucleic acid molecule is present in a sample. In one example, the methods include contacting a sample with at least one oligonucleotide detection probe (for example, a detectably labeled oligonucleotide) that specifically binds to a first target sequence of the target nucleic acid molecule, at least one bifunctional oligonucleotide including a portion that specifically binds to a second target sequence of the target nucleic acid molecule and a portion that specifically binds to an anchor (such as an addressable anchor), and at least one surface comprising at least one anchor (such as an addressable anchor) immobilized on the surface. In some examples, the first target sequence and the second target sequence are contiguous sequences of the target nucleic acid molecule. The sample is contacted with at least one detection probe, at least one bifunctional oligonucleotide, and at least one surface including at least one anchor under conditions sufficient for the detection probe to specifically bind to the first target sequence and for the bifunctional oligonucleotide to specifically bind to the second target sequence and the anchor, such that the target nucleic acid molecule, the detection probe, and the bifunctional oligonucleotide are directly or indirectly bound to (or "tethered" to) the anchor.

According to an aspect of the invention there is provided a method of detecting the presence of a target nucleic acid molecule in a sample, comprising:
a) contacting the sample with:
   i) at least one oligonucleotide detection probe comprising a detectable label,
      wherein the detection probe specifically binds to a first target sequence of the target nucleic acid molecule, optionally wherein the detection probe comprises 15 to 50 nucleotides or 20 to 30 nucleotides;
   ii) at least one bifunctional oligonucleotide that comprises:
      A) a target-specific portion, which specifically binds to a second target sequence of the target nucleic acid molecule that is contiguous to the first target sequence; and
      B) an anchor-specific portion, which specifically binds to an anchor; and
   iii) at least one surface comprising the anchor, which is immobilized on the surface; under conditions sufficient for:
      I) the detection probe to specifically bind to the first target sequence;
      II) the target-specific portion of the bifunctional oligonucleotide to specifically bind to the second target sequence; and
      III) the anchor to specifically bind to the anchor-specific portion of the bifunctional oligonucleotide, such that the target nucleic acid, the detection probe, and the bifunctional oligonucleotide are directly or indirectly bound to the surface via the anchor, thereby producing a tethered target nucleic acid, a tethered detection probe, and a tethered bifunctional oligonucleotide,
      wherein one or more of steps i), ii), and iii) may occur contemporaneously or sequentially;
b) adding a ligase to the tethered detection probe and the tethered bifunctional oligonucleotide, thereby producing a ligated detection probe and bifunctional oligonucleotide;
c) adding a reagent to specifically remove substantially all tethered target nucleic acid molecule, wherein the reagent has substantially no specific activity to remove the ligated detection probe and bifunctional oligonucleotide;
d) removing substantially all unligated detection probe; and
e) detecting on the at least one surface the presence of the detectable label at the position of the anchor, thereby detecting presence of the target nucleic acid molecule in the sample.

The tethered detection probe and bifunctional oligonucleotide are linked or combined (such as by ligation or crosslinking) and then a reagent that specifically removes substantially all of the tethered target nucleic acid molecule is added to the sample; however, the reagent does not remove the linked detection probe-bifunctional oligonucleotide. Substantially all unlinked (e.g., unligated) detection probe is removed (for example, by washing and/or diffusion) and the presence of the detectable label of the detection probe is detected (for example, at the position of the addressable anchor), thereby detecting the presence and/or amount of the target nucleic acid in the sample.

In some embodiments, the methods include detecting the presence and/or amount of a nucleotide variant in a target nucleic acid molecule. In some examples, the methods are substantially as described above, except that the sample is contacted with at least two different bifunctional oligonucleotides, one of which specifically binds to the wild type nucleotide(s) in the target nucleic acid at a variant nucleotide position (VNP) and one of which binds to the variant nucleotide(s) in the target nucleic acid at the VNP. In some examples, the VNP is present in the second target sequence at a position no more than three nucleotides from the junction of the contiguous first and second target sequences in the target nucleic acid. In the presence of a match (for example, specific binding of a variant bifunctional oligonucleotide with a target nucleic acid molecule including the variant nucleotide), the detection probe and the bifunctional oligonucleotide will be tethered (and subsequently ligated), and the detectable label of the detection probe will be detected (for example, following treatment with a reagent that removes substantially all of the tethered but unligated target nucleic acid), indicating presence of the variant nucleotide in the target nucleic acid molecule. In the presence of a mismatch (for example, a variant bifunctional oligonucleotide with a target nucleic acid molecule including the wild type nucleotide), the detection probe and the bifunctional oligonucleotide will not be tethered (and will not subsequently be ligated) in substantial amounts and the detection probe will not be detected (for example, following treatment with a reagent that removes substantially all of the tethered but unligated target nucleic acid), indicating absence of the variant nucleotide in the target nucleic acid molecule.

Also disclosed herein are methods of detecting presence and/or amount of a target nucleic acid molecule in a sample that include contacting the sample with at least one detection probe, at least one bifunctional oligonucleotide, and at least one surface including at least one immobilized anchor (such as an addressable anchor), but that do not include ligation of the detection probe and bifunctional oligonucleotide , and optionally may include treatment with a reagent that removes single-stranded nucleic acids or cleaves nucleic acids including at least one mismatch (such as digestion with a single-strand specific nuclease (such as S1 nuclease) or a mismatch-specific nuclease). Such methods can include contacting the sample with at least one detection probe that specifically binds to a first target sequence in the target nucleic acid molecule; at least one bifunctional oligonucleotide that includes a portion (a target-specific portion) that specifically binds to a second target sequence in the target nucleic acid molecule and a portion (an anchor-specific portion) that specifically binds to an anchor; and at least one surface that includes at least one anchor (such as an addressable anchor) immobilized on the surface, under conditions sufficient for the detection probe to specifically bind to the first target sequence, the target-specific portion of the bifunctional oligonucleotide to specifically bind to the target nucleic acid, and the anchor-specific portion of the bifunctional oligonucleotide to specifically bind to the anchor.

As a result of the specific binding of the detection probe to the target nucleic acid molecule and the bifunctional oligonucleotide to the target nucleic acid and the anchor, each of the target nucleic acid, detection probe, and bifunctional oligonucleotide are tethered (directly or indirectly) to the anchor. The detectable label in the detection probe is then detected on the surface (for example, at the position of the addressable anchor), thereby detecting presence of the target nucleic acid molecule in the sample. In some examples, the methods further include adding a reagent that specifically removes single-stranded nucleic acids and/or cleaves nucleic acids at a mismatch, followed by detection of the detectable label in the detection probe on the surface (for example, at the position of the addressable anchor), thereby detecting presence of the target nucleic acid molecule in the sample. If the detection probe and/or the bifunctional oligonucleotide are exactly complementary to the first target sequence and/or the second target sequence, no cleavage will occur and the detection probe will remain indirectly tethered to the anchor subsequently be detected. However, if the detection probe and/or the bifunctional oligonucleotide are not exactly complementary to the first target sequence and/or the second target sequence, the labeled portion of the detection probe will be cleaved and released, and thus, not detected.

The foregoing and other features of the disclosure will become more apparent from the following detailed description, which proceeds with reference to the accompanying figures.

### BRIEF DESCRIPTION OF THE DRAWINGS

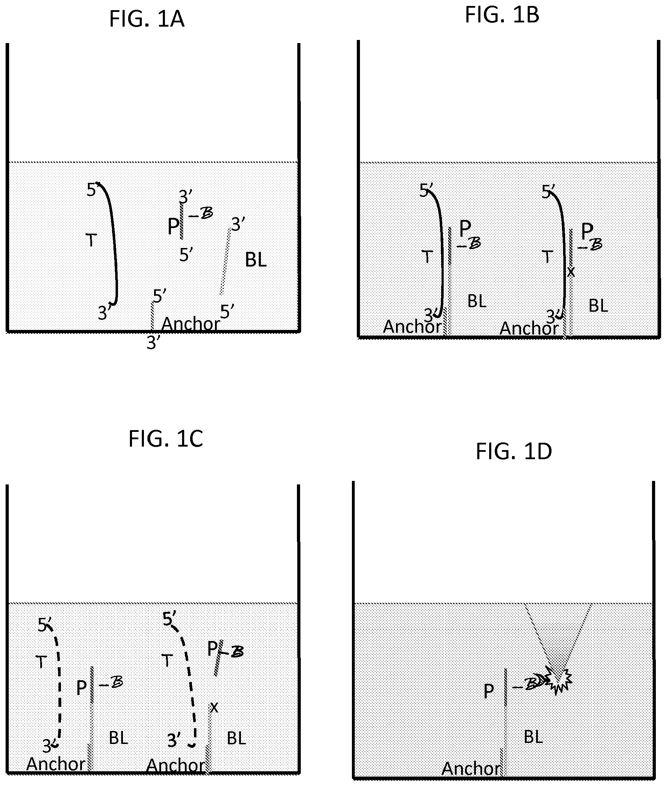

FIGS. 1A-1D are a series of schematic diagrams illustrating an exemplary direct detect ligation assay for detecting wild type and/or variant target nucleic acid in a sample. FIG. 1A shows the combination in solution of a sample (target nucleic acid, T), a biotin-labeled (B) detection probe (P), and a bifunctional oligonucleotide (BL) in a well including an anchor (Anchor) attached to a surface (such as an addressable anchor). The bifunctional oligonucleotide includes a target-specific portion that specifically binds to the target nucleic acid and an anchor-specific portion that specifically binds to the anchor. The detection probe specifically binds to a portion of the target sequence that is contiguous to the portion of the target sequence that is specifically bound by the target-specific portion of the bifunctional oligonucleotide. Hybridization is allowed to occur as shown in FIG. 1B, where the bifunctional oligonucleotide hybridizes to both the anchor and the target nucleic acid and the detection probe hybridizes to the target. This results in a mixture where the target nucleic acid, the detection probe, and the bifunctional oligonucleotide are all directly or indirectly tethered to the anchor. The mixture is then incubated with a ligase. If the target nucleic acid is complementary to the 3' nucleotide(s) of the bifunctional oligonucleotide, the detection probe and bifunctional oligonucleotide will be ligated. If there is a mismatch at or near the 3' end of the bifunctional oligonucleotide (X), the detection probe and bifunctional oligonucleotide will not be ligated. Similarly, if there is a mismatch at or near the 5' end of the detection probe, the detection probe and the bifunctional oligonucleotide will not be ligated (not shown). Following ligase treatment, the mixture is treated with a reagent that removes substantially all of the target nucleic acid (and other non-target nucleic acids in the sample) but does not remove the ligated detection probe-bifunctional oligonucleotide. Unligated detection probe will be washed away if the hybridized RNA is digested by the reagent (FIG. 1C). As shown in FIG. ID, the sample is then incubated with detection reagents (such as avidin-peroxidase and substrate) and signal from the detection probe is detected.

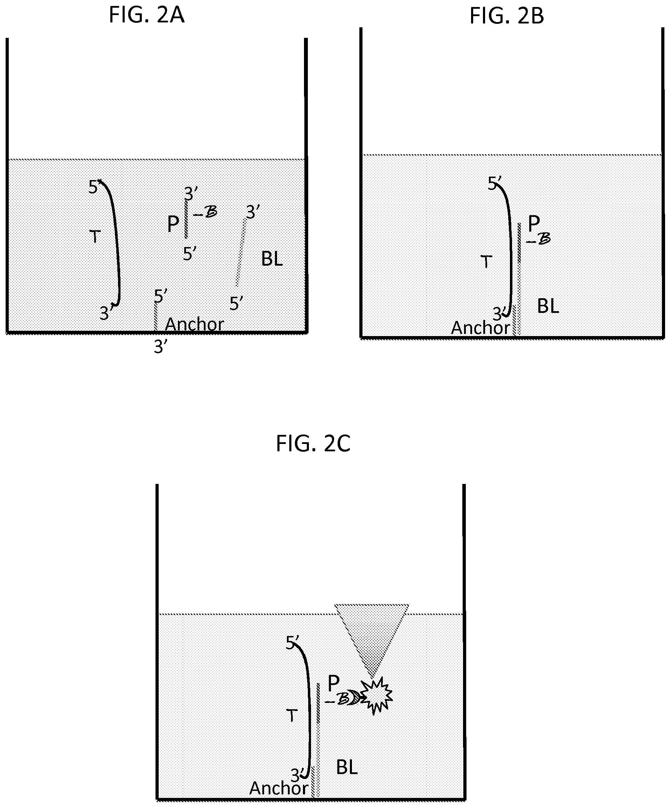

FIGS. 2A-2C are a series of schematic diagrams illustrating an exemplary direct capture and detection method for detecting a target nucleic acid in a sample. FIG. 2A shows the combination in solution of a sample (target nucleic acid, T), a biotin-labeled (B) detection probe (P), and a bifunctional oligonucleotide (BL) in a well including an anchor (Anchor) attached to a surface (such as an addressable anchor). The bifunctional oligonucleotide includes a target-specific portion that specifically binds to the target nucleic acid and an anchor-specific portion that specifically binds to the anchor. The detection probe specifically binds to a portion of the target sequence that is contiguous to (or near) the portion of the target sequence that is specifically bound by the target-specific portion of the bifunctional oligonucleotide. Hybridization is allowed to occur as shown in FIG. 2B, where the bifunctional oligonucleotide hybridizes to both the anchor and the target nucleic acid and the detection probe hybridizes to the target nucleic acid. As shown in FIG. 2C, the sample is then incubated with detection reagents (such as avidin-peroxidase and substrate) and signal from the detection probe is detected.
FIGS. 3A and 3B are a pair of graphs showing average signal intensity at varying *in vitro* transcript (IVT) concentrations for a BRAF V600 wild type detection probe (FIG. 3A) or BRAF V600E detection probe (FIG. 3B), demonstrating specificity of the assay.
FIGS. 4A and 4B are a pair of graphs showing signal intensity at varying ratios of BRAF V600 wild type and V600E IVTs detected in the same well. The data are shown as average signal intensity (FIG. 4A) or average signal intensity as percentage of maximum signal (FIG. 4B).
FIGS. 5A and 5B are a pair of graphs showing signal intensity at varying ratios of BRAF V600 wild type and V600K IVTs detected in the same well. The data are shown as average signal intensity (FIG. 5A) or average signal intensity as percentage of maximum signal (FIG. 5B).
FIGS. 6A and 6B are a pair of graphs showing signal intensity at varying ratios of BRAF V600 wild type and V600E2/D IVTs detected in the same well. The data are shown as average signal intensity (FIG. 6A) or average signal intensity as percentage of maximum signal (FIG. 6B).
FIG. 7 is a graph showing average signal intensity of V600 wild type and V600E detection probes detected in lysates from 6000 cells/well of the indicated melanoma cell lines.
FIG. 8 is a graph showing average signal intensity of V600 wild type and V600E detection probes detected in lysates from 50,000 cells/well of the indicated cell lines.
FIG. 9 is a graph showing average signal intensity of V600 wild type (gray bars) and V600E (black bars) detection probes in FFPE samples from metastatic (met) or primary melanomas.
FIG. 10 is a graph showing average signal intensity of V600 wild type (gray bars) and V600E (black bars) detection probes in clinical melanoma FFPE samples at 0.25 cm² section/well (normalized to BRAF control).

### SEQUENCE LISTING

The nucleic acid sequences listed herein or in the sequence listing submitted herewith are shown using standard letter abbreviations for nucleotide bases, as defined in 37 C.F.R. 1.822. Only one strand of each nucleic acid sequence is shown, but the complementary strand is understood as included by any reference to the displayed strand.
SEQ ID NOs: 1-5 are nucleic acid sequences of BRAF V600 wild type and variant nucleic acids.
SEQ ID NO: 6 is the nucleic acid sequence of an exemplary BRAF V600 detection probe.
SEQ ID NOs: 7-11 are the nucleic acid sequences of target-specific portions of exemplary V600 wild type and variant bifunctional oligonucleotides.

### DETAILED DESCRIPTION

### I. Terms

Unless otherwise noted, technical terms are used according to conventional usage. Definitions of common terms in molecular biology may be found in Benjamin Lewin, Genes VII, published by Oxford University Press, 2000 (ISBN 019879276X); Kendrew et al. (eds.), The Encyclopedia of Molecular Biology, published by Blackwell Publishers, 1994 (ISBN 0632021829); Robert A. Meyers (ed.), Molecular Biology and Biotechnology: a Comprehensive Desk Reference, published by Wiley, John & Sons, Inc., 1995 (ISBN 0471186341); and George P. Rédei, Encyclopedic Dictionary of Genetics, Genomics, and Proteomics, 2nd Edition, 2003 (ISBN: 0-471-26821-6).

The following explanations of terms and methods are provided to better describe the present disclosure and to guide those of ordinary skill in the art to practice the present disclosure. The singular forms "a," "an," and "the" refer to one or more than one, unless the context clearly dictates otherwise. For example, the term "comprising a cell" includes single or plural cells and is considered equivalent to the phrase "comprising at least one cell." As used herein, "comprises" means "includes." Thus, "comprising A or B," means "including A, B, or A and B," without excluding additional elements. Although methods and materials similar or equivalent to those described herein can be used to practice or test the disclosed technology, suitable methods and materials are described below. The materials, methods, and examples are illustrative only and not intended to be limiting.

To facilitate review of the various embodiments of this disclosure, the following explanations of specific terms are provided:
**Anchor:** An oligonucleotide that is attached to or associated with a surface or substrate. In some examples, the anchor is associated with a surface or substrate at an identifiable location, and may be referred to as an "addressable" anchor. In some examples, an oligonucleotide anchor includes a nucleic acid sequence that does not bind (for example, does not specifically bind) to any nucleic acid sequence native to the sample or other non-anchor-specific oligonucleotides included in the methods disclosed herein (such as a detection probe or the target-specific portion of a bifunctional oligonucleotide). In some examples, an anchor does not bind to (for example, does not specifically bind to) and does not have any meaningful similarly (for example less than 10% complementarity) to any human DNA or RNA.
**Complementary:** A nucleic acid molecule is said to be complementary to another nucleic acid molecule if the two molecules share a sufficient number of complementary nucleotides (for example, A-T, A-U, or G-C) to form a stable duplex or triplex when the strands bind (hybridize) to each other, for example by forming Watson-Crick, Hoogsteen, or reverse Hoogsteen base pairs. Stable or specific binding occurs when a nucleic acid molecule (*e.g.,* a detection probe or bifunctional oligonucleotide) remains detectably bound to another nucleic acid (*e.g.,* a target nucleic acid or an anchor) as a result of base pairing between complementary nucleotides in the nucleic acid molecules under the required conditions.
   Complementarity is the degree to which bases in one nucleic acid molecule (*e.g.,* a detection probe nucleic acid molecule) base pair with the bases in a second nucleic acid molecule (*e.g*., target nucleic acid molecule). Complementarity is conveniently described by percentage, that is, the proportion of nucleotides that form base pairs between two molecules or within a specific region or domain of two molecules. For example, if 10 nucleotides of a 15 contiguous nucleotide region of a detection probe nucleic acid molecule form base pairs with a target nucleic acid molecule, that region of the detection probe nucleic acid molecule is said to have 66.67% complementarity to the target nucleic acid molecule. In some examples herein, two nucleic acid molecules (such as a target nucleic acid and a detection probe or a target nucleic acid and the target-specific portion of a bifunctional oligonucleotide) are 100% complementary, while in other examples, two nucleic acid molecules are at least 80% complementary (for example, at least 85%, at least 90%, at least 95%, at least 98%, or more complementary).
**Conditions sufficient for:** Any environment that permits the desired activity, for example, that permits specific binding or hybridization between two nucleic acid molecules (such as between a detection probe and a target nucleic acid or between a bifunctional oligonucleotide and a target nucleic acid and/or anchor) or that permits an enzymatic activity (such as ligase activity or nuclease activity).
**Contact:** Placement in direct physical association; includes both in solid and liquid form. For example, contacting can occur *in vitro* with a nucleic acid detection probe and biological sample in solution.
**Contiguous:** Nucleic acids or sequences that are adjacent (*e.g*., directly adjacent) to one another, for example sharing an edge or boundary or neighboring one another. In some examples, contiguous nucleic acids or sequences are connected to one another (for example without a break). In other examples, contiguous nucleic acids or sequences are directly adjacent, but are not connected to one another (though they are capable of being joined or connected, for example by the activity of a ligase). Contiguous nucleic acids are typically non-overlapping, but in some examples may overlap (for example, overlap by 1, 2, 3, or more nucleotides).
**Detect:** To determine if an agent (such as a signal, particular nucleotide, nucleic acid molecule, and/or organism) is present or absent. In some examples, this can further include quantification. For example, use of the disclosed methods and detection probes in particular examples permits determination of presence, amount, and/or identity of a nucleic acid (such as a target nucleic acid) in a sample.
**Detectable label:** A compound or composition that is conjugated directly or indirectly to another molecule (such as a nucleic acid molecule or a nucleotide) to facilitate detection of that molecule. Specific, non-limiting examples of labels include fluorescent and fluorogenic moieties, chromogenic moieties, haptens, affinity tags, and radioactive isotopes. The label can be directly detectable (e.g., optically detectable) or indirectly detectable (for example, via interaction with one or more additional molecules that are in turn detectable). Exemplary labels in the context of the detection probes disclosed herein are described below. Methods for labeling nucleic acids, and guidance in the choice of labels useful for various purposes, are discussed, *e.g.,* in Sambrook and Russell, in Molecular Cloning: A Laboratory Manual, 3rd Ed., Cold Spring Harbor Laboratory Press (2001) and Ausubel et al., in Current Protocols in Molecular Biology, Greene Publishing Associates and Wiley-Intersciences (1987, and including updates).
**Hybridization:** The ability of complementary single-stranded DNA, RNA, or DNA/RNA hybrids to form a duplex molecule (also referred to as a hybridization complex). Nucleic acid hybridization techniques can be used to form hybridization complexes between a nucleic acid probe (such as a detection probe), and the nucleic acid it is designed to target.
   "Specifically hybridizable" and "specifically complementary" are terms that indicate a sufficient degree of complementarity such that stable and specific binding occurs between the oligonucleotide (or its analog) and the nucleic acid target (such as a DNA or RNA target, such as mRNA or miRNA). The oligonucleotide (such as the detection probe or bifunctional oligonucleotide) need not be 100% complementary (for example, it can be at least 90% complementary, such as at least 91%, at least 92%, at least 93%, at least 94%, at least 95%, at least 96%, at least 97%, at least 98%, or at least 99% complementary) to its target sequence (such as the target nucleic acid or anchor) to be specifically hybridizable; however, in some examples, the oligonucleotide is 100% complementary to a target sequence. In some examples, specific hybridization is also referred to herein as "specific binding."
   Hybridization conditions resulting in particular degrees of stringency will vary depending upon the nature of the hybridization method and the composition and length of the hybridizing nucleic acid sequences. Generally, the temperature of hybridization and the ionic strength (such as the Na⁺ concentration) of the hybridization buffer will determine the stringency of hybridization. Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11).
**Ligate:** Joining together two nucleic acid molecules by a phosphodiester bond between a 3' hydroxyl group of one nucleic acid molecule and a 5' phosphate group of a second nucleic acid molecule. An enzyme that catalyzes the formation of the phosphodiester bond between juxtaposed 5' phosphate and 3' hydroxyl termini of nucleic acids is referred to as a ligase. Ligases include T4 DNA ligase, T7 DNA ligase, thermostable DNA ligase, T3 RNA ligase, and T4 RNA ligase.
**Melting temperature (Tₘ):** Also known as TM₅₀. The temperature at which half of the nucleic acid molecules in a mixture are double-stranded and half of the nucleic acid molecules are single-stranded. In some examples, for example when referring to an oligonucleotide, the Tₘ is the temperature at which 50% of the oligonucleotide and its complement are in a duplex. Methods for determining the Tₘ for DNA or RNA are known to one of ordinary skill in the art.
**Nuclease:** An enzyme that cleaves a phosphodiester bond. An endonuclease is an enzyme that cleaves an internal phosphodiester bond within a nucleotide chain (in contrast to exonucleases, which cleave a phosphodiester bond at the end of a nucleotide chain). Endonucleases include restriction endonucleases or other site-specific endonucleases (which cleave DNA at sequence specific sites), DNase I, S1 nuclease, CEL I nuclease, Mung bean nuclease, Ribonuclease A (RNAse A), Ribonuclease T1 (RNAse T1), Ribonuclease H (RNAse H), RNase I, RNase PhyM, RNase U2, RNase CLB, micrococcal nuclease, and apurinic/apyrimidinic endonucleases. Exonucleases include exonuclease III, exonuclease VII, and Bal 31 nuclease. In particular examples herein, a nuclease is an RNA-specific nuclease, such as RNAse A, RNAse H, or RNAse T1.
**Oligonucleotide:** A plurality of nucleotides joined by phosphodiester bonds, for example between about 6 and about 300 nucleotides in length. An oligonucleotide analog refers to moieties that function similarly to oligonucleotides but have non-naturally occurring portions. For example, oligonucleotide analogs can contain non-naturally occurring portions, such as altered sugar moieties or inter-sugar linkages, such as a phosphorothioate oligodeoxynucleotide.
   Particular oligonucleotides and oligonucleotide analogs can include linear sequences up to about 200 nucleotides in length, for example a detection probe or bifunctional oligonucleotide that is at least 6 nucleotides, for example at least 8, at least 10, at least 15, at least 20, at least 21, at least 25, at least 30, at least 35, at least 40, at least 45, at least 50, at least 100 or even at least 200 nucleotides long, or from about 20 to about 125 nucleotides, about 6 to about 50 nucleotides, or about 10-25 nucleotides, such as 12, 15 or 20 nucleotides, can be obtained, for example from (or complementary to) a target nucleic acid. Oligonucleotides can be produced by methods known to one of skill in the art, including, but not limited to amplification or direct synthesis.
**Probe:** A nucleic acid molecule (for example, an oligonucleotide) capable of hybridizing with a target nucleic acid molecule (*e.g.,* a target DNA or RNA (such as mRNA) nucleic acid molecule) and includes a moiety that is capable of being detected either directly or indirectly (*e.g.,* a detectable label). Thus, probes permit the detection, and in some examples quantification, of a target nucleic acid molecule, such as an mRNA. In some examples, a probe may be referred to herein as a "detection probe."
**Sample:** A biological specimen containing DNA (for example, genomic DNA or cDNA), RNA (including mRNA or short non-coding RNA, such as miRNA), protein, or combinations thereof, obtained from a subject. Examples include, but are not limited to cells, cell lysates, chromosomal preparations, peripheral blood, urine, saliva, tissue biopsy (such as a tumor biopsy or lymph node biopsy), fine needle aspirate, surgical specimen, bone marrow, amniocentesis samples, and autopsy material. In one example, a sample includes RNA, such as mRNA. In particular examples, samples are used directly (*e.g.,* fresh or frozen), or can be manipulated prior to use, for example, by fixation (*e.g*., using formalin) and/or embedding in wax (such as formalin-fixed paraffin-embedded (FFPE) tissue samples).
**Specific binding:** Binding of an agent substantially or preferentially only to a defined target such as a defined oligonucleotide, DNA, RNA, or portion thereof. Thus, a nucleic acid-specific binding agent binds substantially only to a defined nucleic acid, (such as a target sequence in a target nucleic acid) and does not substantially bind to any other nucleic acid. In some examples, specific binding includes the hybridization of one nucleic acid molecule to another (*e.g.,* a detection probe to a target nucleic acid, a bifunctional oligonucleotide to a target nucleic acid, or a bifunctional oligonucleotide to an anchor). For example, a nucleic acid molecule specifically binds another nucleic acid molecule if a sufficient amount of the nucleic acid molecule forms base pairs or is hybridized to its target nucleic acid molecule to permit detection of that binding. In some examples, a nucleic acid molecule specifically binds to another nucleic acid molecule if the two nucleic acids are at least 90% complementary to one another (such as at least 91%, 92%, 93%, 94%, 95%, 96%, 97%, 98%, 99%, or 100% complementary).
**Subject:** Any multi-cellular vertebrate organism, such as human and non-human mammals (*e.g*., veterinary subjects).
**Surface (or substrate):** Any solid support or material which is insoluble, or can be made insoluble by a subsequent reaction. Numerous and varied solid supports are known to those in the art and include, without limitation, nitrocellulose, the walls of wells of a reaction tray, multi-well plates, tubes (such as microfuge tubes), beads (such as polymer or magnetic beads), membranes, microparticles (such as latex particles), or microfluidic channels or cells. Any suitable material with sufficient porosity to allow access by detector reagents and a suitable surface affinity to immobilize capture reagents (*e.g*., oligonucleotides) is contemplated by this term. For example, the porous structure of nitrocellulose has excellent absorption and adsorption qualities for a wide variety of reagents, for instance, oligonucleotides, such as anchors. Nylon possesses similar characteristics and is also suitable. Microporous structures are useful, as are materials with gel structure in the hydrated state.
   Further examples of useful solid supports include natural polymeric carbohydrates and their synthetically modified, cross-linked or substituted derivatives, such as agar, agarose, cross-linked alginic acid, substituted and cross-linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber; synthetic polymers which may be prepared with suitably porous structures, such as vinyl polymers, including polyethylene, polypropylene, polystyrene, polyvinylchloride, polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides; porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and mixtures or copolymers of the above classes, such as graft copolymers obtained by initializing polymerization of synthetic polymers on a pre-existing natural polymer.
**Target nucleic acid molecule:** A defined region or particular portion of a nucleic acid molecule, for example a DNA or RNA of interest. In an example where the target nucleic acid molecule is a target mRNA, such a target can be defined by its specific sequence or function; by its gene or protein name; or by any other means that uniquely identifies it from among other nucleic acids. Exemplary target nucleic acid molecules, such as mRNA, long non-coding RNAs, and short non-coding RNAs are disclosed herein.
**Tethered:** Connected, either directly or indirectly. In some examples, a nucleic acid (such as a detection probe or a bifunctional oligonucleotide) is directly tethered to another nucleic acid (such as a target nucleic acid or an anchor) by specific binding, for example, hybridization. In other examples, a first nucleic acid is indirectly tethered to a second nucleic acid, for example by directly binding to a third nucleic acid which also directly binds to the second nucleic acid. For example, in the methods disclosed herein, a target nucleic acid is indirectly tethered to an anchor if it specifically binds a bifunctional oligonucleotide and the bifunctional oligonucleotide also specifically binds to the anchor. The interaction between tethered nucleic acid molecules may be reversible. For example, a detection probe is tethered to a bifunctional oligonucleotide when both are specifically bound to the same target nucleic acid, but they are not directly connected. If the detection probe and bifunctional oligonucleotide are connected by a phosphodiester bond or other chemical bond (for example by the action of a ligase or crosslinking agent) they are referred to herein as being ligated or linked (though the ligated or linked nucleic acids may also be tethered to other nucleic acid(s), such as a target nucleic acid and/or anchor).
**Variant nucleotide:** A change or alteration in a nucleic acid sequence, such as change in nucleic acid sequence at one or more bases in a target nucleic acid (including substitution, insertion, duplication, and/or deletion of one or more nucleotides). The nucleotide variant can be those variations (e.g., DNA sequence differences) which are generally found between individuals or different ethnic groups and geographic locations which, while having a different sequence, produce functionally equivalent gene products. The term can also refer to variants in the sequence which can lead to gene products that are not functionally equivalent. The term nucleotide variant also encompasses variations which can be classified as alleles and/or mutations which can produce gene products which may have an altered function, produce no gene product, an inactive gene product, or an active gene product produced at an abnormal rate or in an inappropriate tissue or in response to an inappropriate stimulus. In some non-limiting examples, a nucleotide variant is a single nucleotide variant (SNV) or single nucleotide polymorphism (SNP).

Nucleotide variants can be referred to, for instance, by the nucleotide position(s) at which the variation exists (*e.g*., "variant nucleotide position(s)" or VNP), by the change in nucleic acid or amino acid sequence caused by the nucleotide variation, or by a change in some other characteristic of the nucleic acid molecule or protein that is linked to the variation.

In some examples, alterations of a target nucleic acid sequence (*e.g*., an mRNA) are "associated with" a disease or condition. That is, detection of the target nucleic acid sequence can be used to infer the status of a sample with respect to the disease or condition. For example, the target nucleic acid sequence can exist in two (or more) distinguishable forms, such that a first form correlates with absence of a disease or condition and a second (or different) form correlates with the presence of the disease or condition. The two different forms can be qualitatively distinguishable, such as by nucleotide polymorphisms (for example, a SNV) or mutation, and/or the two different forms can be quantitatively distinguishable, such as by the number of copies of the target nucleic acid sequence that are present in a sample.

### II. Ligation-Mediated Methods of Detecting a Target Nucleic Acid Molecule

Disclosed herein are methods of detecting presence and/or amount of a target nucleic acid molecule. Thus, methods are provided that allow for a determination as to whether or not a target nucleic acid molecule is present in a sample of interest. In some examples, the disclosed methods permit quantitation (including semi-quantitative analysis) of the target nucleic acid molecule in the sample if present. In some examples, the methods include ligation of a detection probe (which can specifically bind or hybridize to a first target sequence of the target nucleic acid molecule) to a bifunctional oligonucleotide that specifically binds to an anchor immobilized on a surface (for example, an addressable anchor).

In some embodiments, the methods include detecting a single target using a single type of anchor immobilized on a surface (a "single target assay") or detecting multiple targets, each assay detecting a single target using a single type of anchor immobilized on a surface (e.g., multiple parallel single target assays). In other examples, the disclosed methods include detecting multiple targets using a single type of anchor with multiple detection methods (such as multiple differently labeled detection probes) followed by signal deconvolution. The methods disclosed herein can also be multiplexed, such as the exemplary embodiments provided herein. For example, in some embodiments, two or more (such as at least 2, at least 3, at least 4, at least 5, at least 10, at least 20, at least 30, at least 40 , at least 50, or at least 100, such as 2, 3, 4, 5, 6, 7, 8, 9, 10, 15, 20, 25, 30, 35, 40, 45, 50, or more) different target nucleic acid molecules (with or without variant nucleotides at one or more positions) can be detected in the same sample or reaction (for example, simultaneously or contemporaneously). For example, a single sample can be contacted with two or more different detection probes and two or more different bifunctional oligonucleotides in the same reaction and the different detection probes can be detected by different labels or their localization to distinguishable addressable anchors. In other embodiments, a target nucleic acid can be detected in two or more (such as at least 2, at least 3, at least 4, at least 5, at least 10, at least 20, at least 30, at least 40 , at least 50, or at least 100, such as 2, 3, 4, 5, 6, 7, 8, 9, 10, 15, 20, 25, 30, 35, 40, 45, 50, or more) different samples simultaneously or contemporaneously (for example, high, medium, or low throughput assays). For example, two or more different samples can be contacted with the same detectable detection probe and a bifunctional oligonucleotide with the same target-specific portion, but with different anchor-specific portions and detected by their localization to distinguishable anchors (such as anchors located at a specific site on a surface (*e.g*., an addressable anchor), or anchors located on a specifically distinguishable surface (*e.g*., an addressable bead)). In still further embodiments, two or more target nucleic acid molecules can be detected in two or more different samples simultaneously or contemporaneously. For example, each of two or more samples can be contacted with the same set of two or more different detection probes and two or more different bifunctional oligonucleotides in separate reactions corresponding to the number of samples and the different detection probes in each of the separate reactions can be detected by different labels or their localization to a set of distinguishable addressable anchors, where each set of addressable anchors is immobilized on a different surface or in a distinguishable region of a surface, such as in different wells in a microtiter plate.

In one example, the methods can include contacting the sample with at least one detection probe that specifically binds to a first target sequence in the target nucleic acid molecule; at least one bifunctional oligonucleotide that includes a portion (a target-specific portion) that specifically binds to a second target sequence in the target nucleic acid molecule and a portion (an anchor-specific portion) that specifically binds to an anchor, and at least one surface that includes at least one anchor immobilized on the surface (such as an addressable anchor), under conditions sufficient for the detection probe to specifically bind to the first target sequence, the target-specific portion of the bifunctional oligonucleotide to specifically bind to the target nucleic acid molecule, and the anchor-specific portion of the bifunctional oligonucleotide to specifically bind to the anchor. In some examples, the sample is suspended in an aqueous solution for contacting with the detection probe, the bifunctional oligonucleotide, the anchor, and the surface (*e.g*., FIG. 1A). In some examples, the first target sequence and the second target sequence are contiguous in the target nucleic acid molecule (for example, are directly adjacent).

In some embodiments, contacting the sample with the detection probe, the bifunctional oligonucleotide, and the surface including the anchor occur contemporaneously (for example simultaneously or substantially simultaneously). In other embodiments, contacting the sample with one or more of the detection probe, bifunctional oligonucleotide, anchor, and/or surface occurs sequentially. In some embodiments, the sample is contacted simultaneously or substantially simultaneously with the at least one detection probe, at least one bifunctional oligonucleotide, and at least one surface including at least one anchor. In other embodiments, the sample is contacted with the at least one detection probe and at least one bifunctional oligonucleotide under conditions sufficient for the detection probe to specifically bind to the first target sequence and for the bifunctional oligonucleotide to specifically bind to the second target sequence, which is subsequently contacted with the surface under conditions sufficient for the bifunctional oligonucleotide to specifically bind to the anchor, resulting in tethering the target nucleic acid, the detection probe, and the bifunctional oligonucleotide to the anchor.

As a result of the specific binding of the detection probe to the target nucleic acid molecule and the bifunctional oligonucleotide to the target nucleic acid molecule and the anchor, each of the target nucleic acid, detection probe, and bifunctional oligonucleotide are tethered (directly or indirectly) to the anchor (*e.g.,* FIG. 1B). A ligase is added and the tethered detection probe and bifunctional oligonucleotide are ligated if the detection probe and the bifunctional oligonucleotide are exactly complementary (100% complementary) to the nucleotides at the junction between the first target sequence and the second target sequence, but are not ligated if they are not exactly complementary (less than 100% complementary) to the nucleotides at the junction between the first and second target sequences. In some examples, the tethered detection probe and bifunctional oligonucleotide are ligated if there are one or more mismatches between the detection probe and the first target sequence and/or the bifunctional oligonucleotide and the second target sequence (such as 1, 2, 3 or 4 mismatches or less than or equal to 5 mismatches), but the detection probe and the bifunctional oligonucleotide are exactly complementary to the nucleotides at the junction between the first target sequence and the second target sequence (*e.g*., exact complementarity to 1, 2, 3, or 4 contiguous nucleotides on one or both sides of the junction, or exact complementarity between 2 and 10 nucleotides on one or both sides of or spanning the junction). A reagent (such as a nuclease) that specifically removes substantially all of the target nucleic acid molecule but that has substantially no activity to remove the ligated detection probe-bifunctional oligonucleotide is added and then substantially all of the unligated detection probe is removed (for example by washing) (*e.g*., FIG. 1C). The ligated detection probe-bifunctional oligonucleotide remain tethered to the anchor on the surface and the detectable label is detected on the surface at the position of the anchor, thereby detecting presence of the target nucleic acid molecule in the sample (*e.g.,* FIG. 1D).

The disclosed methods can also be utilized to detect the presence and/or amount of more than one target nucleic acid molecule in a sample, for example simultaneously or contemporaneously. Thus, the methods can detect at least or up to two (such as at least 3, at least 4, at least 5, at least 10, at least 20, at least 30, at least 40 , at least 50, or at least 100, such as 2, 3, 4, 5, 10, 20, 50, 100, or more) target nucleic acid molecules in a sample. For example for detection of two distinct target nucleic acid molecules, the methods can include contacting the sample with a first detection probe that specifically binds to a first target sequence in a first target nucleic acid molecule and a second detection probe that specifically binds to a first target sequence in a second target nucleic acid molecule. The sample is also contacted with a first bifunctional oligonucleotide that includes a target-specific portion that specifically binds to a second target sequence in the first target nucleic acid molecule and a second bifunctional oligonucleotide that includes a target-specific portion that specifically binds to a second target sequence in the second target nucleic molecule. The first and second bifunctional oligonucleotides each include an anchor-specific portion which specifically binds to distinct anchors immobilized on one or more surfaces (which are also contacted with the sample). Additional target nucleic acids (such as at least, or up to, 3, 4, 5, 10, 20, 50, 100, or more target nucleic acids) can be detected by adding further distinct detection probes, bifunctional oligonucleotides, and anchors to the methods disclosed herein.

Samples of use in the disclosed methods include any specimen that includes nucleic acid (such as genomic DNA, cDNA, viral DNA or RNA, rRNA, tRNA, mRNA, short non-coding RNA (such as miRNA), oligonucleotides, nucleic acid fragments, modified nucleic acids, synthetic nucleic acids, or the like) and are discussed in further detail in Section V, below. In some embodiments, the sample is suspended in an aqueous solution. Exemplary aqueous solutions in which a sample may be suspended include water, buffers (such as phosphate buffered saline), or reaction solutions (such as a lysis buffer and/or high salt buffer, for example, as described in Example 1). In one non-limiting example, the sample is a formalin-fixed paraffin-embedded (FFPE) tissue sample or section and all or a portion of the FFPE sample is suspended in an aqueous solution (such as a buffer, for example a lysis buffer). In other examples, a sample suspended in an aqueous solution includes a cell lysate, a resuspended cell pellet (such as a fixed cell pellet), or isolated nucleic acids.

In some examples, when (or after) the sample is suspended in an aqueous solution, the sample is contemporaneously contacted with the at least one detection probe, at least one bifunctional oligonucleotide, and, in more specific examples, at least one surface including at least one anchor (such as an addressable anchor). As utilized herein, contemporaneously indicates events that occur simultaneously, or substantially simultaneously. For example, if the sample is contacted contemporaneously with the detection probe, the bifunctional oligonucleotide, and the surface including at least one anchor, the detection probe, bifunctional oligonucleotide, and the surface may be mixed or combined prior to addition of the sample. In other examples, one or more of the sample, detection probe, bifunctional oligonucleotide, and surface may be added or mixed together in a series of steps, wherein there is only a brief gap between the additions (for example, less than 10 minutes, less than 5 minutes, or less than 2 minutes between any of contacting the sample with the detection probe, the bifunctional oligonucleotide, and/or the surface). In further examples, contemporaneously contacting may indicate a longer time between additions of any of the detection probe, the bifunctional oligonucleotide, and/or the surface, for example, if the conditions are temporarily not sufficient for specific binding of the detection probe, bifunctional oligonucleotide, and/or the addressable anchor on the surface to their appropriate partner (for example, the conditions are at room temperature or below, such as a temperature at 28°C or below, 27°C or below, 26°C or below, 25°C or below, 24°C or below, 23°C or below, 22°C or below, 21°C or below, or 20°C or below, such as 4°C to 28°C, 20 °C to 25°C, 20°C to 28°C, or 15°C to 25°C).

In other embodiments, the sample is on a solid support, such as a tissue section on a solid support (such as a glass slide). In some examples, a sample on a solid support is contacted with at least one detection probe under conditions sufficient for the detection probe to specifically bind to the first target sequence in the target nucleic acid. The sample (including the detection probe) is then suspended in an aqueous solution and contacted with at least one bifunctional oligonucleotide and at least one surface including at least one anchor (such as at least one addressable anchor) under conditions sufficient for the bifunctional oligonucleotide to specifically bind to the second target sequence in the target nucleic acid and the anchor. In other examples, a sample on a solid support is contacted with at least one detection probe and at least one bifunctional oligonucleotide under conditions sufficient for the detection probe to specifically bind to the first target sequence and the bifunctional oligonucleotide to specifically bind to the second target sequence. The sample (including the detection probe and the bifunctional oligonucleotide) is then suspended in an aqueous solution and contacted with the at least one surface under conditions sufficient for the bifunctional oligonucleotide to specifically bind to the anchor on the surface.

### A. Target Nucleic Acid Molecules

Target nucleic acid molecules of use in the methods disclosed herein include any nucleic acid present in a biological sample, including DNA, RNA, or fragments thereof. In particular embodiments, the target nucleic acid molecule is RNA or a fragment thereof. RNAs that can be detected in the methods disclosed herein include mRNAs (or fragments thereof) and/or non-coding RNAs. Non-coding RNAs include long non-coding RNA, such as non-protein encoding transcripts of about 200 nucleotides or more in length, though some long non-coding RNAs may be shorter than 200 nucleotides long. In some examples, long non-coding RNAs are RNA transcripts that include little or no open reading frame. Non-coding RNAs also include small non-coding RNAs, including microRNA (miRNA), small interfering RNA (siRNA), Piwi-interacting RNA (piRNA), transcription initiation RNA (tiRNA), centromere repeat associated small interacting RNA (crasiRNA), and telomere-specific small RNA (tel-siRNA). Typically, small non-coding RNAs are about 60 nucleotides or less in length, but some may be longer, particularly in an unprocessed form. Additional small non-coding RNAs include small nuclear RNAs (snRNA), which are typically about 150 nucleotides in length and small nucleolar RNAs (snoRNA). Various databases of non-coding RNAs are available on the World Wide Web, for example at lncrnadb.com, biobases.ibch.poznan.pl/ncRNA/, mirbase.org, www-snorna.biotoul.fr, and evolveathome.com/snoRNA/snoRNA.php. One of ordinary skill in the art can identify target nucleic acids, such as mRNA, long non-coding RNA or short non-coding RNAs for detection using the disclosed methods. Other nucleic acid molecules can also be detected using the disclosed methods including DNA (*e.g*., genomic DNA or cDNA) or other RNA (such as rRNA or tRNA).

In some examples, a target nucleic acid molecule includes a v-Raf murine sarcoma viral oncogene homolog B1 (BRAF) nucleic acid, a Kirsten rat sarcoma viral oncogene homolog (KRAS) nucleic acid, an epidermal growth factor receptor (EGFR) nucleic acid, an estrogen receptor nucleic acid, an ALK nucleic acid, an EML nucleic acid, a BRCA1 or BRCA2 nucleic acid, a FoxL2 nucleic acid, a GNAS nucleic acid, a Cldn2 nucleic acid, a Fam120c nucleic acid, a Gprasp1 nucleic acid, a Stard8 nucleic acid, an Apln nucleic acid, an Fmr1 nucleic acid, a Diap2 nucleic acid, a Med14 nucleic acid, or a Ddx26b nucleic acid, or variants of any of the foregoing including one or more mutations at one or more positions.

In some examples, more than one target nucleic acid molecule is detected in the sample, for example simultaneously or contemporaneously. For example at least 2,, at least 5, at least 10, at least 50, at least 100, or up to 500, up to 250, up to 100, or up to 50 different target nucleic acid molecules can be detected in a sample. In some examples, a target nucleic acid molecule is a control or reference target nucleic acid molecule, such as a housekeeping (or endogenous control) nucleic acid molecule or an *in vitro* transcript.

### B. Detection Probes

The disclosed methods include contacting a sample with an oligonucleotide detection probe (which includes a detectable label) that specifically binds to a first target sequence in a target nucleic acid molecule. In additional examples, the methods include contacting the sample with two or more (such as 2, 3, 4, 5, 6, 7, 8, 9, 10, or more) detection probes which each specifically bind to a distinct contiguous portion of the first target sequence. For example, the first target sequence may be about 25 nucleotides in length and the detection probe specifically binds to the first target sequence. Alternatively, the first target sequence may be 50 nucleotides in length and a first detection probe specifically binds to the first 25 nucleotides of the first target sequence and a second detection probe specifically binds to the second 25 nucleotides of the first target sequence. Use of two or more detection probes that bind to contiguous portions of the first target sequence increases the amount of detectable label present for detection, as each detection probe includes a label. In further examples, the methods can include contacting the sample with two or more detection probes that each specifically bind to a distinct target nucleic acid molecule. Such multiplexing methods are discussed in more detail above.

In some examples, the detection probes disclosed herein can be selected to include at least 10, at least 15, at least 20, at least 25, or more consecutive nucleotides complementary to a portion of a target nucleic acid molecule (such as about 6 to 75, 10 to 60, 15 to 50, 18 to 45, 20 to 42, or 23 to 41 consecutive nucleotides complementary to a target nucleic acid molecule). In some examples, the detection probe is 100% complementary to the first target sequence; however 100% complementarity is not necessarily required for specific binding. In some examples, the detection probe is at least 90%, at least 91%, at least 92%, at least 93%, at least 94%, at least 95%, at least 96%, at least 97%, at least 98%, at least 99, or 100% complementary to the first target sequence. Particular lengths of detection probes that can be used to practice the methods of the present disclosure include detection probes having at least 6, 10, 15, 20, 25, 30, 35, 40, 45, 50, or more contiguous nucleotides that specifically bind to (for example, are complementary to) a target nucleic acid molecule. In a particular non-limiting example, a detection probe used in the disclosed methods is 12 to 75 (such as 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75) nucleotides in length. In some examples, a detection probe may be about 15-50, about 20-45, about 20-40, about 20-35, about 20-30, about 15-40, about 15-35, about 15-30, or about 15-25 nucleotides in length. In one particular example, the detection probe is 25 nucleotides in length.

Conditions resulting in specific binding of the detection probe to the target nucleic acid will vary depending upon the methods utilized and the composition and length of the detection probe. Generally, the temperature of hybridization and the ionic strength (such as the Na⁺ concentration) of the hybridization buffer will determine the stringency of hybridization. In one non-limiting example, specific binding (such as specific binding of the detection probe to the target nucleic acid molecule and/or specific binding of the bifunctional oligonucleotide to the target nucleic acid molecule and/or the anchor) includes binding (such as hybridization) that occurs at about 37°C for about 16-24 hours.

In some examples, the detection probes utilized in the disclosed methods have a melting temperature (Tₘ, the temperature at which half of the nucleic acid molecules in a mixture are double-stranded and half of the nucleic acid molecules are single-stranded) of at least about 23°C, such as at least about 37°C, at least about 42°C, at least about 45°C, at least about 50°C, at least about 55°C, at least about 60°C, at least about 65°C, at least about 70°C, at least about 75°C, or at least about 80°C, such as about 23°C to 70°C (for example, about 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70°C). In one example, the detection probes utilized in the disclosed methods have a Tₘ of about 60°C to about 70°C. In a particular non-limiting example, the detection probes utilized in the disclosed methods have a Tₘ of about 60°C. In some examples, the detection probes have a Tₘ of about 59°C to about 62°C (such as about 59.1, 59.2, 59.3, 59.4, 59.5, 59.6, 59.7, 59.8, 59.9, 60.0, 60.1, 60.2, 60.3, 60.4, 60.5, 60.6, 60.7, 60.8, 60.9, 61.0, 61.1, 61.2, 61.3, 61.4, 61.5, 61.6, 61.7, 61.8, or 61.9°C). Methods of calculating the Tₘ of a probe are known to one of ordinary skill in the art (see *e.g.,* Sambrook et al., Molecular Cloning: A Laboratory Manual, 3d ed., Cold Spring Harbor Press, 2001, Chapter 10). Tools for calculating the Tₘ of a probe are also available on the World Wide Web (such as at promega.com/techserv/tools/biomath/calc11.htm). In some embodiments, the "base-stacking" method is used to calculate probe Tₘ. In some examples (for example, if multiple detection probes are utilized in a single reaction), the detection probes are selected to each have the same or a similar Tₘ in order to facilitate simultaneous detection of non-variant and/or variant nucleotides in a sample. For example, the detection probes may be selected to have a Tₘ within at least 2.5°C of one another (such as within 2.4°C, 2.3°C, 2.2°C, 2.1°C, 2.0°C, 1.9°C, 1.8°C, 1.7°C, 1.6°C, 1.5°C, 1.4°C, 1.3°C, 1.2°C, 1.1°C, 1.0°C, 0.9°C, 0.8°C, 0.7°C, 0.6°C, 0.5°C, 0.4°C, 0.3°C, 0.2°C, 0.1°C, or less of one another).

In particular examples, the detection probe includes a 5' phosphate moiety. The 5' phosphate is included to facilitate ligation of a detection probe to the bifunctional oligonucleotide (for example, when the 5' end of the detection probe is adjacent to and ligated to the 3' end of a bifunctional oligonucleotide). Synthesis of oligonucleotides (for example by direct chemical synthesis or amplification from a template) produces oligonucleotides lacking a 5' phosphate. Thus, in some examples, a detection probe is modified to include a 5' phosphate prior to use in the disclosed methods. In some examples, the detection probe is 5' phosphorylated with a polynucleotide kinase, such as T4 polynucleotide kinase.

The oligonucleotide detection probes disclosed herein also include a detectable label. Detectable labels are discussed in detail in Section IV, below. The detectable label can be attached to the detection probe at any location, so long as it does not interfere with specific binding of the detection probe to the target nucleic acid and it does not interfere with ligation of the detection probe to the bifunctional oligonucleotide. Thus, in some examples, the detection probe is end-labeled (for example, 5' end-labeled or 3' end-labeled). The detection probe can also be internally labeled with the detectable label, for example by incorporation of one or more labeled nucleotides (such as biotin-labeled dT) in the detection probe. One of ordinary skill in the art can determine whether a detectable label interferes with specific binding of the detection probe to the target nucleic acid and/or ligation of the detection probe to the bifunctional oligonucleotide.

### C. Bifunctional Oligonucleotides

The disclosed methods also utilize at least one bifunctional oligonucleotide that includes two portions, a target-specific portion and an anchor-specific portion. The target-specific portion specifically binds to a second target sequence in the target nucleic acid molecule. In particular examples, the second target sequence is contiguous in the target nucleic acid molecule with the first target sequence. The anchor-specific portion specifically binds to an anchor which is immobilized on a surface (for example, an addressable anchor). In further examples, the methods can include contacting the sample with two or more bifunctional oligonucleotides that each include a distinct target-specific portion and a distinct anchor-specific portion. Such multiplexing methods are discussed in more detail above.

Particular lengths of bifunctional oligonucleotides that can be used to practice the methods of the present disclosure include bifunctional oligonucleotides having at least 12, 15, 20, 25, 30, 35, 40, 45, 50, 55, 60, 65, 70, 75, 80, 85, 90, 95, 100, 125, 150, 175, 200, or more contiguous nucleotides, including a portion that specifically binds to the target nucleic acid molecule (target-specific portion) and a portion that specifically binds to an anchor (anchor-specific portion). In some examples, the bifunctional oligonucleotide is 100% complementary to the second target sequence; however 100% complementarity is not necessarily required for specific binding. In some examples, the portion of the bifunctional oligonucleotide that specifically binds the target nucleic acid (the target-specific portion) is at least 90%, at least 91%, at least 92%, at least 93%, at least 94%, at least 95%, at least 96%, at least 97%, at least 98%, at least 99, or 100% complementary to the second target sequence. In some examples, the portion of the bifunctional oligonucleotide that specifically binds to the anchor is at least 90%, at least 91%, at least 92%, at least 93%, at least 94%, at least 95%, at least 96%, at least 97%, at least 98%, at least 99, or 100% complementary to the anchor sequence.

In a particular non-limiting example, a bifunctional oligonucleotide used in the disclosed methods is 12 to 20 nucleotides in length. In some examples, the target-specific portion of the bifunctional oligonucleotide may be about 12-100 nucleotides (such as about 15-75, about 25-75, about 30-75, about 35-75, about 40-75, about 45-75, about 50-100, or about 50-75 nucleotides in length). In particular examples, the target-specific portion of the bifunctional oligonucleotide is 25-30 nucleotides (such as 25, 26, 27, 28, 29, or 30 nucleotides). In some examples, the anchor-specific portion of the bifunctional oligonucleotide may be about 12-100 nucleotides (such as about 15-75, about 25-75, about 30-75, about 35-75, about 40-75, about 45-75, about 50-100, or about 50-75 nucleotides in length). In one particular example, the anchor-specific portion of the bifunctional oligonucleotide is 25 nucleotides long. The target-specific portion and the anchor-specific portion of the bifunctional oligonucleotide may be the same length, or they may be different lengths.

In some examples, the target-specific portion of the bifunctional oligonucleotides utilized in the disclosed methods has a melting temperature of at least about 23°C, such as at least about 37°C, at least about 42°C, at least about 45°C, at least about 50°C, at least about 55°C, at least about 60°C, at least about 65°C, at least about 70°C, at least about 75°C, or at least about 80°C, such as about 23°C to 70°C (for example, about 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or 70°C). In one example, the target-specific portion of the bifunctional oligonucleotides utilized in the disclosed methods have a Tₘ of about 60°C to about 70°C. In a particular non-limiting example, the target-specific portion of the bifunctional oligonucleotides have a Tₘ of about 60°C.

In some examples, the target-specific portion and the anchor-specific portion of the bifunctional oligonucleotide are contiguous. In other examples, the target-specific portion and the anchor-specific portion of the bifunctional oligonucleotide are separated, for example, by a spacer. The spacer may be included to reduce steric hindrance in the binding of the target-specific portion to the target nucleic acid and the binding of the anchor-specific portion to the anchor. In some examples the spacer is a series of nucleotides, such as about 5 to 50 nucleotides (for example, about 10-50, about 15-40, about 20-30, or about 25 nucleotides long). In other examples, the spacer is a series of carbon atoms, such as about 6 to 36 carbon atoms (for example, about 6-30, about 12-30, about 12-24, or about 18 carbon atoms). In additional examples, the spacer can be a mixture of nucleotides and carbon atoms. In further examples, the spacer may include peptide nucleic acids. If the spacer includes nucleotides, the nucleotide sequence of the spacer is selected such that it does not specifically bind to any nucleic acid in the sample under the conditions of the reaction(s) in the disclosed methods. For example, if the target nucleic acid is a human nucleic acid, the spacer oligonucleotide sequence is selected to have no significant homology to any human nucleic acid (for example, the human transcriptome, if the target nucleic acid is an mRNA) or to have substantially no specific binding to any human nucleic acid.

In particular examples, the bifunctional oligonucleotide includes a 5' phosphate moiety. The 5' phosphate is included to facilitate ligation of a bifunctional oligonucleotide to a detection probe (for example, when the 5' end of the bifunctional oligonucleotide is adjacent to and ligated to the 3' end of a detection probe). Synthesis of oligonucleotides (for example by direct chemical synthesis or amplification from a template) produces oligonucleotides lacking a 5' phosphate. Thus, in some examples, a bifunctional oligonucleotide is modified to include a 5' phosphate prior to use in the disclosed methods. In some examples, the bifunctional oligonucleotide is 5' phosphorylated with a polynucleotide kinase, such as T4 polynucleotide kinase.

### D. Anchors and Surfaces

The disclosed methods include at least one surface with at least one anchor immobilized on the surface (for example on an array, bead, or flow cell). In further examples, the methods can include contacting the sample with a surface with two or more distinct immobilized anchors or with two or more surfaces each with a distinct immobilized anchor. Such multiplexing methods are discussed in more detail above. In particular examples, one or more anchors are immobilized on a surface (such as a substrate, bead, or flow cell) at a distinguishable location, providing one or more addressable anchors.

In some examples, an anchor is an oligonucleotide of about 8 to 150 nucleotides in length (for example, about 15 to 100, 20 to 80, 25 to 75, or 25 to 50, such as about 15, 20, 25, 30, 35, 40, 45, 50, 55, 60, 65, 70, 75, 80, 85, 90, 95, 100, 110, 120, 130, 140, or 150 nucleotides). In one non-limiting example, the anchor is about 25 nucleotides in length. In some examples, the anchor is 100% complementary to the anchor-specific portion of a bifunctional oligonucleotide; however 100% complementarity is not necessarily required for specific binding. In some examples, the anchor that binds to the bifunctional oligonucleotide is at least 90%, at least 91%, at least 92%, at least 93%, at least 94%, at least 95%, at least 96%, at least 97%, at least 98%, at least 99, or 100% complementary to the anchor-specific portion of the bifunctional oligonucleotide.

In some examples, the anchor includes a first portion that specifically binds to the anchor-specific portion of the bifunctional oligonucleotide and a second portion that acts as a spacer between the surface and the first portion of the anchor. In some examples, the second portion of the anchor is about 6 to 60 carbon atoms or nucleotides in length (such as about 6, 7, 8, 9, 10, 11, 12, 24, 30, 36, 42, 48, 54, or 60 carbon atoms or nucleotides). In particular examples, the spacer between the surface and the portion of the anchor that specifically binds to the anchor-specific portion of the bifunctional oligonucleotide is a 7 carbon spacer.

In some examples, the sequence of the anchor (and thus, the complementary anchor-specific portion of the bifunctional oligonucleotide) is selected so that the anchor does not bind (for example, does not specifically bind) to any nucleic acid in the sample, the detection probe, or the target-specific portion of the bifunctional oligonucleotide. Appropriate anchor sequences can be identified by *in silico* methods, empirical methods, or a combination thereof. For example, candidate anchor sequences can be compared to nucleic acids in the genome or transcriptome of the organism from which the sample is derived (for example with BLAST, BLAT, or other available software). Candidate anchor sequences that have any meaningful similarity (complementarity) to any nucleic acid from the organism are eliminated from consideration for use as anchors and candidate anchor sequences that do not have any meaningful similarity to any nucleic acid sequence from the organism are selected as anchors. In some examples, the selected anchor sequences are empirically tested for binding to detection probes and/or nucleic acids from the organism in the presence and absence of the corresponding bifunctional oligonucleotide and any that produce detectable signal in the absence of the bifunctional oligonucleotide are also eliminated from use as anchors. Thus, in some examples the anchor has no more than 10% complementarity to a region of at least 100 contiguous nucleotides (such as no more than 5%, 4%, 3%, 2%, or 1% complementarity) or no more than 1% complementarity to a region of at least 1000 contiguous nucleotides (such as no more than 0.05%, 0.04%, 0.03%, 0.02%, or 0.01% complementarity) of a human DNA or RNA sequence.

In some embodiments, the disclosed methods include one or more surfaces that include multiple distinct anchors (for example, two or more distinct addressable anchors). Thus, in some examples, it is desirable to develop a set of anchors which are substantially dissimilar from one another. Methods of designing and synthesizing anchors or a set of anchors are described *e.g.,* in PCT Publication No. WO 98/24098.

The anchors are referred to herein in some examples as "addressable" anchors. This term is intended to indicate that the anchor immobilized on a surface in such a way that it is located at a spatially discrete and identifiable position (for example, its location can be reliably and consistently determined on the surface, or population of surfaces). In some examples, an addressable anchor is immobilized in a single spatially discrete region on a surface that has multiple regions (which in some examples each include a distinct immobilized addressable anchor). For example, an addressable anchor may be present (immobilized at) a spatially discrete region of a well of a reaction tray, which may also include distinct addressable anchors at distinct spatially discrete regions of the well. In other examples, the addressable anchor is immobilized on a single spatially discrete surface which can be part of a population of multiple spatially discrete surfaces that each includes a distinct immobilized addressable anchor. For example, addressable anchors can be immobilized on a population of beads, wherein subpopulations of the beads each include a distinct immobilized addressable anchor (such as a bead array). In further examples, addressable anchors can be immobilized on a population of micro fluidic channels, wherein each channel of the population includes a distinct immobilized addressable anchor (for example, a microfluidic chip).

In some embodiments, the disclosed methods include at least one surface with at least one addressable anchor immobilized on the surface. Some of the surfaces (or substrates) which can be used in the disclosed methods are readily available from commercial suppliers. In some embodiments, the surface is a 96-, 384-, or 1536-well microtiter plate, such as modified plates sold by Corning Costar or BD Biosciences (for example, gamma-irradiated plates). In other embodiments, a substrate includes one or more beads (such as a population of beads that can be differentiated by size or color, for example by flow cytometry). In some embodiments, a substrate includes a flow cell (such as a flow cell or a microfluidic ship with a plurality of channels). Alternatively, a surface comprising wells which, in turn, comprise indentations or "dimples" can be formed by micromachining a substance such as aluminum or steel to prepare a mold, then microinjecting plastic or a similar material into the mold to form a structure. Alternatively, a structure comprised of glass, plastic, ceramic, or the like, can be assembled. The separator can be, for example, a piece of material, *e.g.,* silicone, with holes spaced throughout, so that each hole will form the walls of a test well when the three pieces are joined. The subdivider can be, for example, a thin piece of material, *e.g.,* silicone, shaped in the form of a screen or fine meshwork. In some examples, the base is a flat piece of material (for example glass or plastic), in, for example, the shape of the lower portion of a typical microplate used for a biochemical assay. The top surface of the base can be flat, or can be formed with indentations that will align with the subdivider shape to provide full subdivisions, or wells, within each sample well. The three pieces can be joined by standard procedures, for example the procedures used in the assembly of silicon wafers.

Suitable materials for the surface include, but are not limited to: glass, silica, gold, silver, a gel or polymer, nitrocellulose, polypropylene, polyethylene, polybutylene, polyisobutylene, polybutadiene, polyisoprene, polyvinylpyrrolidine, polytetrafluroethylene, polydimethylsiloxane, polyvinylidene difluoride, polyfluoroethylene-propylene, polyethylenevinyl alcohol, polymethylpentene, polycholorotrifluoroethylene, polysulfones, hydroxylated biaxially oriented polypropylene, aminated biaxially oriented polypropylene, thiolated biaxially oriented polypropylene, ethyleneacrylic acid, thylene methacrylic acid, and blends of copolymers thereof (see U.S. Patent No. 5,985,567).

In general, suitable characteristics of the material that can be used to form the surface include: being amenable to surface activation such that upon activation, the surface of the support is capable of covalently (or irreversibly) attaching a biomolecule such as an anchor thereto; amenability to *"in situ"* synthesis of biomolecules; being chemically inert such that at the areas on the support not occupied by a biomolecule (such as an addressable anchor) are not amenable to non-specific binding, or when non-specific binding occurs, such materials can be readily removed from the surface without removing the oligonucleotides or proteins.

A wide variety of array formats for arrangement of the anchors can be employed in accordance with the present disclosure. One suitable format includes a two-dimensional pattern of discrete cells (such as 4096 squares in a 64 by 64 array). As is appreciated by one of ordinary skill in the art, other array formats including, but not limited to slot (rectangular) and circular arrays are equally suitable for use (see U.S. Patent No. 5,981,185). In some examples, the array is a multi-well plate.

In one embodiment, preformed nucleic acid anchors, such as oligonucleotide anchors, can be situated on or within the surface of a test region by any of a variety of conventional techniques, including photolithographic or silkscreen chemical attachment, disposition by ink jet technology, capillary, screen or fluid channel chip, electrochemical patterning using electrode arrays, contacting with a pin or quill, or denaturation followed by baking or UV-irradiating onto filters (see, *e.g.,* Rava et al. (1996). U.S. Patent No. 5,545,531; Fodor et al. (1996). U.S. Patent No. 5,510,270; Zanzucchi et al. (1997). U.S. Patent No. 5,643,738; Brennan (1995). U.S. Patent No. 5,474,796; PCT WO 92/10092; PCT WO 90/15070). Anchors can be placed on top of the surface of a test region or can, for example in the case of a polyacrylamide gel pad, be embedded within the surface in such a manner that some of the anchor protrudes from the surface and is available for interactions with an oligonucleotide, such as a bifunctional oligonucleotide.

In one embodiment, preformed oligonucleotide anchors are derivatized at the 5' end with a free amino group; dissolved at a concentration routinely determined empirically (e.g., about 1 µM) in a buffer such as 50 mM phosphate buffer, pH 8.5 and 1 mM EDTA; and distributed with a Pixus nanojet dispenser (Cartesian Technologies) in droplets of about 10.4 nanoliters onto specific locations within a test well whose upper surface is that of a fresh, dry DNA Bind plate (Corning Costar). In another embodiment, preformed oligonucleotide anchors are derivatized at the 3' end with a free amino group and include a 7 carbon spacer. Anchor oligonucleotides are dissolved at 20 µM in 0.5 M Phosphate buffer at pH 8.5 and are contact printed on Falcon 1172 plates, gamma irradiated (BD Biosciences) using capillary pins in a humidified chamber. Depending on the relative rate of oligonucleotide attachment and evaporation, it may be required to control the humidity in the wells during preparation.

In another embodiment, oligonucleotide anchors can be synthesized directly on the surface of a test region, using conventional methods such as, for example, light-activated deprotection of growing oligonucleotide chains (for example, in conjunction with the use of a site directing "mask") or by patterned dispensing of nanoliter droplets of deactivating compound using a nanojet dispenser. Deprotection of all growing oligonucleotides that are to receive a single nucleotide can be done, for example, and the nucleotide then added across the surface. In another embodiment, oligonucleotide anchors are attached to the surface via the 3' ends of the oligonucleotides, using conventional methodology.

### F. Contacting Sample with Detection Probe/Bifunctional Oligonucleotide/Surface

The disclosed methods include contacting a sample with at least one detection probe, at least one bifunctional oligonucleotide, and at least one surface including at least one anchor under conditions sufficient for the detection probe to specifically bind to the target nucleic acid and for the bifunctional oligonucleotide to specifically bind to the target nucleic acid and the anchor on the surface. In some examples, the disclosed methods are multiplexed and the sample is contacted with a plurality of detection probes, a plurality of bifunctional oligonucleotides, and/or a surface including a plurality of addressable anchors or a plurality of surfaces each comprising a distinct addressable anchor. For example, a sample from a subject, such as a human subject, can be analyzed for the presence of two or more target nucleic acid molecules simultaneously or contemporaneously, by using distinct detection probes and bifunctional oligonucleotides specific for each target of interest and distinct addressable anchors specific for each bifunctional oligonucleotide. In other examples, at least two different samples, for example from at least two different subjects, can be analyzed for the presence of one or more target nucleic acid molecules simultaneously or contemporaneously.

Among the parameters which can be varied are salt concentration, buffer, pH, temperature, time of incubation, and amount and type of denaturant such as formamide. Typically, the conditions sufficient for specific binding include incubation of the sample, at least one detection probe, at least one bifunctional oligonucleotide and at least one surface with at least one anchor at about 37°C or higher (such as about 37°C, 42°C, 50°C, 55°C, 60°C, 65°C, 70°C, 75°C, or higher). In some examples the incubation is about 35-50°C (such as about 37-50°C, about 37-45°C, or about 37-42°C). In one example, the incubation is at about 37°C. In some examples, the nucleic acids in the sample are denatured (for example at about 95°C to about 105°C for about 5-15 minutes) and incubated with at least one detection probe, at least one bifunctional oligonucleotide, and at least one surface with at least one anchor for between about 10 minutes and about 72 hours (for example, at least about 1 hour to 48 hours, about 6 hours to 24 hours, about 12 hours to 18 hours, or overnight) at a temperature ranging from about 4°C to about 70°C (for example, about 37°C to about 65°C, about 42°C to about 60°C, or about 50°C to about 60°C). In one example, the at least one detection probe, at least one bifunctional oligonucleotide, and at least one surface with at least one anchor are incubated at 37°C for 16-24 hours (such as overnight).

The detection probe and/or the bifunctional oligonucleotide can be added to the sample and/or the surface at a concentration ranging from about 10 pM to about 10 nM (such as about 30 pM to 5 nM, about 100 pM to about 1 nM). If the methods include a plurality of detection probes and bifunctional oligonucleotides, each detection probe and bifunctional oligonucleotide is added to the sample and/or surface at a concentration ranging from about 10 pM to about 10 nM (such as about 30 pM to 5 nM, about 100 pM to about 1 nM). In some examples, one or more detection probes are added to the sample and/or the surface at a concentration of 10 pM to 1 nM each (such as about 100 pM to 1 nM, for example about 167 pM). In some examples, one or more bifunctional oligonucleotides are added to the sample and/or the surface at a concentration of about 1 pM to 5 nM each (such as about 10 pM to 1 nM, about 20 pM to 100 pM, for example about 30 pM).

The incubation can be in any suitable buffer that permits the specific binding of the detection probe to the target nucleic acid and the specific binding of the bifunctional oligonucleotide to the target nucleic acid and the anchor. In some examples, the buffer is a cell lysis buffer, such as the lysis buffer described below. In other examples, the buffer is a hybridization buffer. In further examples, the buffer is a modified buffer, such as a mixture of lysis buffer and high salt buffer (as described in Example 1). In some examples the modified buffer is utilized to increase or optimize the signal obtained in the detection step of the assay. In particular examples, the salt concentration of the buffer is about 150 mM to 1 M (such as about 200 mM to 1 M, about 300 mM to 1 M, about 400 mM to 1 M, or about 500 mM to 1 M). In some examples, the buffer includes about 150 mM to 1 M NaCl, for example, about 450 mM NaCl or 1 M NaCl. In additional examples, the formamide concentration of the buffer is about 8-25% (such as about 10-25%, about 10-20%, or about 15-20%). In particular examples, the buffer includes about 10% or 20% formamide.

### G. Ligation of Detection Probe and Bifunctional Oligonucleotide

In particular embodiments, following the incubation of the sample with the at least one detection probe, at least one bifunctional oligonucleotide, and at least one surface including at least one anchor (such as an addressable anchor), producing a tethered target nucleic acid, a tethered detection probe and tethered bifunctional oligonucleotide (directly or indirectly bound to the anchor), a ligase is added (for example, under conditions sufficient for ligation of two oligonucleotides), producing a ligated detection probe-bifunctional oligonucleotide. One of ordinary skill in the art can select an appropriate ligase for use in the disclosed methods. For example, if the detection probe and bifunctional oligonucleotide are DNA oligonucleotides, the ligase is a DNA ligase, such as T4 DNA ligase, T3 DNA ligase, or a thermostable DNA ligase (such as Taq DNA ligase). If the detection probe and bifunctional oligonucleotide are RNA oligonucleotides, the ligase is an RNA ligase, such as T3 RNA ligase or T4 RNA ligase. In other examples, chemical "click" ligation may be used; however, this method of ligation may not allow discrimination of wild type or variant target nucleic acids (for example, using the methods discussed below).

The ligation reaction is carried under conditions sufficient to produce a ligated detection probe-bifunctional oligonucleotide. In some examples, the surface is washed prior to the ligation reaction (for example, to remove untethered nucleic acid molecules and to replace the solution with a ligation buffer). In some examples, the mixture including the tethered detection probe and the tethered bifunctional oligonucleotide is incubated with T4 DNA ligase at about 16-42°C (such as about 16-37°C, 22-37°C, 20-25°C, 32-37°C, or room temperature) for about 30 minutes to 18 hours (such as about 1, 2, 3, 4, 5, 6, 7, 8, 9, 10, 11, 12, 13, 14, 15, 16, 17, or 18 hours). In a particular example, the mixture including the tethered detection probe and the tethered bifunctional oligonucleotide is incubated with T4 DNA ligase for 2 hours at 37°C. In some examples, the ligation is carried out in a ligase buffer (such as a commercially available ligase buffer, for example the ligase buffer provided with New England Biolabs catalog number M0202). In particular examples, the ATP concentration of the buffer is reduced (for example, compared to commercially available ligase buffer) in order to optimize ligation, for example ligation of a DNA detection probe and DNA bifunctional oligonucleotide on an RNA target nucleic acid molecule template. In some examples, the final ATP concentration present in the ligase reaction is less than 1 mM (for example about 1-100 µM, about 1-10 µM, about 2-20 µM, about 5-50 µM, or about 1, 2, 3, 4, 5, 6, 7, 8, 9, or 10 µM). In one particular example, the ligation reaction includes a final ATP concentration of about 2 µM.

### H. Removal of Target Nucleic Acid

Following ligation of the detection probe and bifunctional oligonucleotide, a reagent that specifically removes substantially all of the tethered target nucleic acid molecule (such as target nucleic acid molecules that are indirectly tethered to the anchor) is added. In some examples, the reagent may also remove substantially all non-tethered target nucleic acid molecules. Non-tethered target nucleic acid molecules may also be removed by washing. The reagent is selected such that is does not substantially remove the ligated detection probe-bifunctional oligonucleotide or the anchor. In particular examples, the reagent is an RNA-specific nuclease (for example, when the target nucleic acid is RNA and both the detection probe and bifunctional oligonucleotide are DNA). In some examples, the RNA-specific nuclease is capable of digesting single-stranded RNA. In other examples, the RNA-specific nuclease is capable of digesting RNA in an RNA-DNA hybrid. Exemplary RNA-specific nucleases include RNAse A, RNAse H, and RNAse T1. In one particular example, RNAse H is added following ligation of the detection probe and bifunctional oligonucleotide. In other examples, the reagent is a nuclease that can recognize mismatches and can cleave a target nucleic acid molecule (such as a DNA target nucleic acid molecule) at the site of the mismatch. Thus, in one example, S1 nuclease is added following ligation of the detection probe and bifunctional oligonucleotide.

The reaction is carried under conditions sufficient to remove substantially all of the tethered target nucleic acid molecule without substantially removing the ligated detection probe-bifunctional oligonucleotide. In some examples, the mixture including the tethered target nucleic acid and the ligated detection probe-bifunctional oligonucleotide is incubated with RNAse H at about 22-50°C (such as about 22-37°C, 25-40°C, or 32-37°C) for about 30 minutes to 18 hours (such as about 1, 2, 3, 4, 5, 6, 7, 8, 9, 10, 11, 12, 13, 14, 15, 16, 17, or 18 hours). In a particular example, the mixture including the tethered target nucleic acid and the ligated detection probe-bifunctional oligonucleotide is incubated with RNAse H for 1 hour at 37°C. In some examples, the reaction is carried out in a nuclease buffer (such as a commercially available nuclease buffer). In another example, the nuclease buffer is that described in Example 1. In one example, the nuclease buffer is added to the ligation reaction mixture, without washing or removing the ligation reaction buffer.

### I. Removal of Unligated Detection Probe

Prior to detection, in some examples, substantially all unligated detection probe present on the surface or in solution in contact with the surface is removed. As discussed above, detection probes that are specifically bound to the first target sequence in the target nucleic acid (and which do not include any mismatches with the first target sequence at or near the junction between the first target sequence and the second target sequence in the target nucleic acid) will be ligated to the bifunctional oligonucleotide that is specifically bound to the second target sequence of the target nucleic acid. The ligated detection probe-bifunctional oligonucleotide is indirectly tethered to the surface through specific binding of the anchor-specific portion of the bifunctional oligonucleotide to the anchor. Detection probes with a mismatch with the target nucleic acid at or near the junction (for example, no more than ten, no more than 9, no more than 8, no more than 7, no more than 6, no more than 5, no more than 4, no more than 3, no more than 2, or no more than 1 nucleotide from the junction) will not be ligated and are tethered to the surface only indirectly through specific binding of the detection probe to the target nucleic acid, which is tethered to the surface through specific binding to the bifunctional oligonucleotide, which is in turn specifically bound to the anchor immobilized on the surface. Therefore, unligated detection probe will be released into solution when the target nucleic acid is substantially removed (for example by the nuclease treatment step described above).

Unligated detection probe can be removed by one or more washes of the surface. One of ordinary skill in the art can select appropriate wash conditions to remove substantially all of the unligated detection probe (and other non-tethered nucleic acids present in the solution), without substantially removing the tethered ligated detection probe-bifunctional oligonucleotide.

### J. Detection of Target Nucleic Acid

The presence of the target nucleic acid in the sample is determined by detecting presence of the detectable label, for example at the position of the anchor. That is, the target nucleic acid is indicated to be present in the sample if the detectable label is detected at the position of the anchor (such as at the position of an addressable anchor), while the target nucleic acid is indicated to be absent from the sample if the detectable label is not detected (for example below background levels) at the position of the anchor.

An appropriate detection method is selected based on the particular detectable label included in the detection probe. In some examples, the presence of the detectable label is determined by a suitable colorimetric assay, wherein presence of the label results in a colored product. In other examples, the presence of the detectable label is determined by detection of fluorescence (for example if the detectable label is a fluorescent label) or chemiluminescence. In particular examples, an addressable anchor is immobilized in an array (such as a microarray) and the label is detected using a microarray imager. Microarray imagers are commercially available and include OMIX, OMIX HD, CAPELLA, or SUPERCAPELLA imagers (HTG Molecular Diagnostics, Tucson, Arizona), TYPHOON imager (GE Life Sciences, Piscataway, NJ), GENEPIX microarray scanner (Molecular Devices, Sunnyvale, CA), or GENECHIP scanner (Affymetrix, Santa Clara, CA). In other examples, an anchor is immobilized on a bead and the label is detected by flow cytometry or related methods (such as utilizing a LUMINEX 200 or FLEXMAP 3D (Luminex Corporation, Austin, TX), or other suitable instrument). One of ordinary skill in the art can select an appropriate detection system based on the particular label(s) and assay format.

In one non-limiting example, the detectable label is biotin. Methods of detecting biotin-labeled nucleic acids are well known in the art. In some examples, the detection probe (which is ligated to the bifunctional oligonucleotide and tethered to the anchor) is contacted with avidin or streptavidin (SA) conjugated to an enzyme such as horseradish peroxidase (HRP) or alkaline phosphatase (AP). An enzyme substrate (for example, a chemiluminescent substrate or a chromogenic substrate) is added and chemiluminescence or colored product is detection. In some examples, methods to increase or amplify the signal are utilized to increase sensitivity of the assay. For example, streptavidin conjugated to an HRP polymer (SA-polyHRP) can be utilized to increase the signal produced. PolyHRP conjugates are commercially available, for example SA-PolyHRP20, SA-PolyHRP40, or SA-PolyHRP80 (Fitzgerald Industries International, Acton, MA).

### K. Detection of Variant Nucleotides in Target Nucleic Acid

In particular embodiments of the methods disclosed herein, the presence and/or amount of a nucleotide variant in a target nucleic acid can be detected. As discussed above, the presence of a mismatch between the detection probe and the first target sequence in the target nucleic acid will prevent ligation of the detection probe to the bifunctional oligonucleotide. Similarly, presence of a mismatch between the bifunctional oligonucleotide and the second target sequence in the target nucleic acid will prevent ligation of the detection probe to the bifunctional oligonucleotide. In either situation, the unligated detection probe is ultimately removed and is not detected. Thus, if there is a variant nucleotide in the target nucleic acid, particularly a variant at or near the junction between the first and second target sequences, the target nucleic acid will not be detected, though it is present in the sample.

In some examples, the presence and/or amount of a variant nucleotide(s) (or wild type nucleotide(s)) can be detected by including at least two bifunctional oligonucleotides in the assay, wherein one of the bifunctional oligonucleotides specifically binds to wild type nucleotide(s) at or near the junction of the first and second target sequences and one of the bifunctional oligonucleotides specifically binds to variant nucleotide(s) at or near the junction of the first and second target sequences. Specific binding of the wild type or variant bifunctional oligonucleotide permits ligation of the detection probe and bifunctional oligonucleotide and ultimately detection of the probe. Use of addressable anchors (which are specifically bound by the anchor-specific portion of the bifunctional oligonucleotide) permit discrimination of the wild type and variant target nucleic acids in a single reaction (for example, in a single well), for example by utilizing distinct addressable anchors (and thus distinct anchor-specific portions of the bifunctional oligonucleotide) for the wild type and variant targets.

In other examples, the presence and/or amount of a variant nucleotide(s) (or wild type nucleotide(s)) can be detected by including at least two detection probes in the assay, wherein one of the detection probes specifically binds to wild type nucleotide(s) at or near the junction of the first and second target sequences and one of the detection probes specifically binds to variant nucleotide(s) at or near the junction of the first and second target sequences. Specific binding of the wild type or variant detection probe permits ligation of the detection probe and bifunctional oligonucleotide and ultimately detection of the probe. In this example, the detection probes can be discriminated in a single reaction (such as a single well) by including distinguishable detectable labels on the wild type and variant detection probes. Alternatively, if the detection probes include the same detectable label, they can be detected in separate reactions (such as separate wells).

Thus, in some examples, a position of at least one nucleotide in the second target sequence is a wild type nucleotide or a variant nucleotide. The position of the wild type/variant nucleotide is referred to as a variant nucleotide position (VNP). The VNP is located at or near the junction between the contiguous first and second target sequences in the target nucleic acid. The VNP is located at a position in the target nucleic acid such that if the detection probe (or bifunctional oligonucleotide) does not specifically bind to the target nucleic acid, it will substantially prevent ligation of the detection probe and bifunctional oligonucleotide. In some examples, the VNP is the nucleotide at the junction of the contiguous first and second target sequences. In other examples, the VNP is no more than ten nucleotides from the junction (for example, more than 9, no more than 8, no more than 7, no more than 6, no more than 5, no more than 4, no more than 3, no more than 2, or no more than 1 nucleotide from the junction). In some embodiments, the detection probe (or the bifunctional oligonucleotide) is 100% complementary to the wild type or variant sequence of the target nucleic acid over the entire detection probe (or bifunctional oligonucleotide). However, so long as the detection probe (or bifunctional oligonucleotide) is exactly complementary to the wild type or variant nucleotide(s) at the VNP, some lesser degree of complementarity over the remainder of the detection probe (or bifunctional oligonucleotide) is possible, so long as the detection probe (or bifunctional oligonucleotide) specifically binds to a target sequence including the wild type or variant nucleotide(s), as appropriate.

The disclosed methods can be used to detect any type of nucleotide variant (for example, substitution, insertion, duplication, and/or deletion of one or more nucleotides), so long as detection probes or bifunctional oligonucleotides complementary to the variant and wild type (non-variant) VNP can be designed and synthesized. The VNP may be part of a protein coding sequence and may result in an alteration in one or more amino acids encoded by a nucleic acid sequence (for example, produces one or more amino acid substitutions, insertions, or deletions) or may be a "silent" change, such as a nucleotide variant which does not result in an alteration of the amino acid sequence. The VNP may also be in a non-coding region of a nucleotide, including but not limited to an untranslated region or an intron. In some examples, the VNP includes a substitution of one or more nucleotides (such as 2, 3, or more nucleotides) as compared to the wild type sequence, a deletion of one or more nucleotides (such as 2, 3, or more) as compared to the wild type sequence, an insertion of one or more nucleotides (such as 2, 3, or more) as compared to the wild type sequence, and/or a duplication of one or more nucleotides (such as 2, 3, or more) as compared to the wild type sequence.

In particular examples, the VNP is present in the second target sequence of the target nucleic acid. A sample is contacted with at least one detection probe that specifically binds to a first target sequence that is contiguous with the second target sequence in the target nucleic acid; at least two bifunctional oligonucleotides, one of which specifically binds to the wild type nucleotide(s) at the VNP and the other of which specifically binds to the variant nucleotide(s) at the VNP; and at least at least two distinct anchors (which may be immobilized on the same surface (for example at addressable locations) or on distinct surfaces). The at least two bifunctional oligonucleotides also include distinct anchor-specific portions, which each specifically bind to one of the two distinct anchors. The ligation, removal of target nucleic acid, washing, and detection of the detectable label are all as described above. If a mixture of variant and wild type target nucleic acids is present in the sample, both the wild type and variant bifunctional oligonucleotides will be ligated to the detection probe in amounts proportional to the amounts of the corresponding non-variant and variant target nucleic acids in the sample. Thus, in some examples, the disclosed methods are semi-quantitative or quantitative.

Exemplary variants that can be detected using the methods disclosed herein are shown in Table 1. One of ordinary skill in the art can identify additional variants of interest, including those associated with various diseases and disorders. For example, variants associated with cancer are available on the World Wide Web at cancer.sanger.ac.uk/cancergenome/projects/census.

**Table 1. Exemplary variants**

| **Gene** | **Reference Sequence** | **Variant Nucleotide Position (Amino acid change)** |
|---|---|---|
| KRAS | NM_004985 | 216 G>T (G12V) |
| KRAS | NM_004985 | 216 G>A (G12D) |
| KRAS | NM_004985 | 215 G>T (G12C) |
| KRAS | NM_004985 | 219 G>A (G13D) |
| KRAS | NM_004985 | 363 A>T (Q61L) |
| KRAS | NM_004985 | 364 A/C; 364 A>T (Q61H) |
| BRAF | NM_004333 | 1860 T>A (V600E); 1860 TG>AA (V600E2); 1859 GT>AA (V600K); 1860 TG>AT (V600D) |
| EGFR | NM_005228 | 2527 G>T (D761Y) |
| EGFR | NM_005228 | 2615 C>T (T790M) |
| EGFR | NM_005228 | 2819 T>G (L858R) |
| ER | NM_000125 | 1142 A>G (K303R) |
| FoxL2 | NM_023067 | 820 C>G |
| GNAS | NM_ 000516 | 960C>T |
| Cldn2 | MGSCv37 X chr | 136339038 C>G |
| Fam120c | MGSCv37 X chr | 147900720 C>T |
| Gprasp1 | MGSCv37 X chr | 132327321 T>C |
| Stard8 | MGSCv37 X chr | 96257763 A>G |
| Apln | MGSCv37 X chr | 45380062 G>A |
| Fmr1 | MGSCv37 X chr | 65969007 A>C |
| Diap2 | MGSCv37 X chr | 126995483 G>C |
| Med14 | MGSCv37 X chr | 12253226 A>T |
| Ddx26b | MGSCv37 X chr | 53749712 A>G |

### III. Direct Detection Methods of Detecting a Target Nucleic Acid

Also disclosed herein are methods of detecting presence and/or amount of a target nucleic acid in a sample that include contacting the sample with at least one detection probe, at least one bifunctional oligonucleotide, and at least one surface including at least one immobilized anchor (such as at least one addressable anchor), (referred to as a "direct detection" method). In some examples, the direct detection methods optionally include a nuclease digestion step, for example, treating the sample with a single-strand specific nuclease (such as S1 nuclease) or a nuclease capable of cleaving a nucleotide mismatch (such as CEL 1 nuclease).

In some embodiments, the methods include contacting the sample with at least one detection probe that specifically binds to a first target sequence in the target nucleic acid; at least one bifunctional oligonucleotide that includes a portion (a target-specific portion) that specifically binds to a second target sequence in the target nucleic acid and a portion (an anchor-specific portion) that specifically binds to an anchor; and at least one surface that includes at least one anchor immobilized on the surface, under conditions sufficient for the detection probe to specifically bind to the first target sequence, the target-specific portion of the bifunctional oligonucleotide to specifically bind to the target nucleic acid, and the anchor-specific portion of the bifunctional oligonucleotide to specifically bind to the anchor. In some examples, the sample is suspended in an aqueous solution for contacting with the detection probe, the bifunctional oligonucleotide, the anchor, and the surface (e.g., FIG. 2A). In some examples, the first target sequence and the second target sequence are contiguous in the target nucleic acid (for example, are directly adjacent). However, in other examples, the first target sequence and the second target sequence are not contiguous in the target nucleic acid. For example, the target nucleic acid may be a contiguous nucleic acid, but the first target sequence may be separated from the second target sequence in the target nucleic acid by one or more nucleotides (such as 1, 5, 10, 25, 50, 75, 100, 150, 200, 250, 300, or more nucleotides). In some examples, the first target sequence and second target sequence in the target nucleic acid molecule are separated by about 1-500 nucleotides, such as about 10-400, about 20-300, about 50-200, about 10-100, about 50-100, about 10-50, or about 25-100 nucleotides.

In some embodiments, contacting the sample with the at least one detection probe, the at least one bifunctional oligonucleotide, and the at least one surface including the at least one anchor occur contemporaneously (for example simultaneously or substantially simultaneously). In other embodiments, contacting the sample with one or more of the detection probe, bifunctional oligonucleotide, and/or surface including at least one anchor occurs sequentially. In some embodiments, the sample is contacted simultaneously or substantially simultaneously with the at least one detection probe, at least one bifunctional oligonucleotide, and at least one surface including at least one anchor. In other embodiments, the sample is contacted with the at least one detection probe and at least one bifunctional oligonucleotide under conditions sufficient for the detection probe to specifically bind to the first target sequence and for the bifunctional oligonucleotide to specifically bind to the second target sequence, which is subsequently contacted with the surface including at least one anchor under conditions sufficient for the bifunctional oligonucleotide to specifically bind to the anchor, resulting in tethering the target nucleic acid, the detection probe, and the bifunctional oligonucleotide to the anchor.

As a result of the specific binding of the detection probe to the target nucleic acid and the bifunctional oligonucleotide to the target nucleic acid and the anchor, each of the target nucleic acid, detection probe, and bifunctional oligonucleotide are tethered (directly or indirectly) to the anchor (*e.g.,* FIG. 2B). The detectable label in the detection probe is then detected on the surface at the position of the addressable anchor, thereby detecting presence of the target nucleic acid in the sample (*e.g.,* FIG. 2C). The direct detection methods and reagents are substantially as described in Section II above, except that in some embodiments the ligation of the detection probe and bifunctional oligonucleotide and the removal of the target nucleic acid steps are omitted.

In other embodiments, the ligation of the detection probe and bifunctional oligonucleotide is omitted; however nuclease digestion is included, for example digestion with a single-strand specific nuclease (*e.g*., S1 nuclease) or a mismatch-specific nuclease (such as CEL I nuclease). Without being bound by theory, it is believed that digestion with a single-strand specific nuclease or mismatch-specific nuclease will cleave a mismatch (or S1-sensitive "bulge" caused by a mismatch) if the detection probe and the bifunctional oligonucleotide are not exactly complementary to the first target sequence and the second target sequence.

Thus, in some examples, the methods include contacting the sample with at least one detection probe that specifically binds to a first target sequence in the target nucleic acid; at least one bifunctional oligonucleotide that includes a portion (a target-specific portion) that specifically binds to a second target sequence in the target nucleic acid and a portion (an anchor-specific portion) that specifically binds to an anchor; and at least one surface that includes at least one anchor immobilized on the surface, under conditions sufficient for the detection probe to specifically bind to the first target sequence, the target-specific portion of the bifunctional oligonucleotide to specifically bind to the target nucleic acid, and the anchor-specific portion of the bifunctional oligonucleotide to specifically bind to the anchor. A single-stand specific nuclease (such as S1 nuclease, RNase I, RNase T1, or RNase A) or a mismatch-specific nuclease (such as, endonuclease V, T4 endonuclease VII, mung bean nuclease, or CEL I nuclease) is added and if the detection probe and/or the bifunctional oligonucleotide are exactly complementary to the first target sequence and/or the second target sequence, no cleavage will occur and the detection probe will remain indirectly tethered to the anchor subsequently be detected. However, if the detection probe and/or the bifunctional oligonucleotide are not exactly complementary to the first target sequence and/or the second target sequence, the labeled portion of the detection probe will be cleaved and released, and thus, not detected.

In some examples, the nuclease digestion is carried under conditions sufficient to remove tethered detection probe that is not exactly complementary to the target nucleic acid, without substantially removing tethered detection probe that is exactly complementary to the target nucleic acid. In some examples, the mixture including the tethered target nucleic acid, detection probe, and bifunctional oligonucleotide is incubated with a nuclease (such as S1 nuclease, RNase I, RNase T1, or RNase A) at about 22-50°C (such as about 30-45°C or about 35-42°C, for example, at about 45°C, about 40°C, about 37°C, about 35°C, about 30°C, or about 25°C) for about 30 minutes to 18 hours (such as about 1, 2, 3, 4, 5, 6, 7, 8, 9, 10, 11, 12, 13, 14, 15, 16, 17, or 18 hours). The amount of nuclease included in the reaction can be determined by one of skill in the art. In a particular example, about 0.1 U/µl to 1 U/µl S1 nuclease is included (such as about 1 U/µl, about 0.5 U/µl, about 0.25 U/µl, or about 0.125 U/µl). One of ordinary skill in the art can vary the reaction conditions, such as time, temperature, amount of nuclease, salt concentration, metal ion concentration, or other factors in order to optimize sensitivity and/or specificity of the methods. In some examples, the reaction is carried out in a nuclease buffer (such as a commercially available nuclease buffer). In another example, the nuclease buffer is that described in Example 1. In one example, the nuclease buffer is added to the initial reaction mixture, without washing or removing the reaction buffer. The presence and/or amount of any target nucleic acid can be detected using the direct detection methods described herein. These methods may also be used to detect variants in a target nucleic acid, such as substitution of one or more nucleotides (such as 2, 3, or more nucleotides) as compared to the wild type sequence, a deletion of one or more nucleotides (such as 2, 3, or more) as compared to the wild type sequence, an insertion of one or more nucleotides (such as 2, 3, or more) as compared to the wild type sequence, and/or a duplication of one or more nucleotides (such as 2, 3, or more) as compared to the wild type sequence. The methods for detection of variants is substantially as described in Section II(K), above, with the omission of the ligation step, as discussed herein. However, in some examples, in the direct detection methods, the VNP can be positioned at or near (for example, within about 1, 2, 3, 4, or 5 nucleotides of) the middle of the detection probe or target-specific portion of the bifunctional oligonucleotide. Without being bound by theory, it is believed that positioning the VNP at or near the middle of the detection probe or target-specific portion of the bifunctional oligonucleotide will provide the greatest challenge to hybridization if a mismatch is present and will also result in cleavage by the nuclease treatment, thereby increasing the specificity and sensitivity of the method.

In additional examples, the direct detection method can be used to detect the presence of a gene fusion in a sample. For example, a sample suspected of having a gene fusion can be contacted with a detection probe that specifically binds to one of the fusion partners (for example on one side of a known or suspected breakpoint) and a bifunctional oligonucleotide with a target-specific portion that specifically binds to the other fusion partner (for example on the other side of a known or suspected breakpoint). The ligation-mediated method described in Section II can also be used to detect gene fusions if the breakpoint (the site of the fusion of the two genes) is known and the detection probe and the bifunctional oligonucleotide can be designed to specifically bind to the gene fusion directly adjacent to one another (to permit ligation). However, because the detection probe and the bifunctional oligonucleotide are not ligated in the direct detection method, they do not have to be directly adjacent when they specifically bind to the target nucleic acid. Thus, this method is particularly useful when the exact breakpoint in a gene fusion is not known, or is variable. Exemplary gene fusions that can be detected using the methods disclosed herein include EML4/ALK fusions, BCR/ABL1 fusions, TMPRSS2/ERG fusions, TMPRSS2/ETV4 fusions, EWSR1/FLI1 fusions, DDX5/PRKCB fusions, CCDC134/ZNF75A fusions, COL3A1/GRSF1 fusions, IREB2/OXR1 fusions, and MYB/NFIB fusions. One of ordinary skill in the art can identify additional gene fusions for detection with the disclosed methods.

### IV. Detectable Labels

The detection probes described herein include one or more detectable labels. Detectable labels are well known in the art. A "detectable label" is a molecule or material that can be used to produce a detectable signal that indicates the presence or concentration of the detection probe (*e.g.,* the bound or hybridized detection probe) in a sample. Thus, a labeled detection probe provides an indicator of the presence or concentration of a target nucleic acid sequence in a sample. The disclosure is not limited to the use of particular labels, although examples are provided.

In some examples, each of the detection probes included in a plurality of detection probes utilized in the disclosed methods are labeled with the same detectable label. In other examples at least one detection probe is labeled with a different detectable label than at least one other detection probe in a plurality of detection probes. In some examples, the plurality of detection probes can include at least 2, 3, 4, 5, 6, 7, 8, 9, 10, or more different detectable labels.

A label associated with one or more nucleic acid molecules (such as an oligonucleotide detection probe) can be detected either directly or indirectly. A label can be detected by any known or yet to be discovered mechanism including absorption, emission and/or scattering of a photon (including radio frequency, microwave frequency, infrared frequency, visible frequency and ultra-violet frequency photons). Detectable labels include colored, fluorescent, phosphorescent and luminescent molecules and materials, catalysts (such as enzymes) that convert one substance into another substance to provide a detectable difference (such as by converting a colorless substance into a colored substance or vice versa, or by producing a precipitate or increasing sample turbidity), haptens, and paramagnetic and magnetic molecules or materials. Additional detectable labels include Raman (light scattering) labels (*e.g.,* NANOPLEX biotags, Oxonica, Bucks, UK).

In non-limiting examples, detection probes are labeled with dNTPs covalently attached to hapten molecules (such as a nitro-aromatic compound (*e.g*., dinitrophenyl (DNP)), biotin, fluorescein, digoxigenin, etc.). Methods for conjugating haptens and other labels to dNTPs (*e.g.,* to facilitate incorporation into labeled probes) are well known in the art. For examples of procedures, see, *e.g.,* U.S. Patent Nos. 5,258,507, 4,772,691, 5,328,824, and 4,711,955. A label can be directly or indirectly attached to a dNTP at any location on the dNTP, such as a phosphate (*e.g.*, α, β or γ phosphate) or a sugar. In some examples, detection of labeled nucleic acid molecules can be accomplished by contacting the hapten-labeled detection probe with a primary anti-hapten antibody. In one example, the primary anti-hapten antibody (such as a mouse anti-hapten antibody) is directly labeled with an enzyme. In another example, a secondary anti-antibody (such as a goat anti-mouse IgG antibody) conjugated to an enzyme is used for signal amplification. In other examples, the hapten is biotin and is detected by contacting the biotin-labeled detection probe with avidin or streptavidin conjugated to an enzyme, such as horseradish peroxidase (HRP) or alkaline phosphatase (AP).

Additional examples of detectable labels include fluorescent molecules (or fluorochromes). Numerous fluorochromes are known to those of ordinary skill in the art, and can be selected, for example from Life Technologies (formerly Invitrogen), *e.g.,* see, The Handbook - A Guide to Fluorescent Probes and Labeling Technologies). Examples of particular fluorophores that can be attached (for example, chemically conjugated) to a nucleic acid molecule (such as a detection probe) are provided in U.S. Patent No. 5,866,366 to Nazarenko et al.*,* such as 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumarin 151); cyanosine; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate (FITC), and QFITC (XRITC); 2', 7'-difluorofluorescein (OREGON GREEN®); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red®); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives.

Other suitable fluorophores include thiol-reactive europium chelates which emit at approximately 617 nm (Heyduk and Heyduk, Analyt. Biochem. 248:216-27, 1997; J. Biol. Chem. 274:3315-22, 1999), as well as GFP, LISSAMINE, diethylaminocoumarin, fluorescein chlorotriazinyl, naphthofluorescein, 4,7-dichlororhodamine and xanthene (as described in U.S. Patent No. 5,800,996 to Lee et al.) and derivatives thereof. Other fluorophores known to those of ordinary skill in the art can also be used, for example those available from Life Technologies (Invitrogen; Molecular Probes (Eugene, OR)) and including the ALEXA FLUOR® series of dyes (for example, as described in U.S. Patent Nos. 5,696,157, 6,130,101 and 6, 716,979), the BODIPY® series of dyes (dipyrrometheneboron difluoride dyes, for example as described in U.S. Patent Nos. 4,774,339, 5,187,288, 5,248,782, 5,274,113, 5,338,854, 5,451,663 and 5,433,896), Cascade Blue (an amine reactive derivative of the sulfonated pyrene described in U.S. Patent No. 5,132,432) and Marina Blue (U.S. Patent No. 5,830,912).

In addition to the fluorochromes described above, a fluorescent label can be a fluorescent nanoparticle, such as a semiconductor nanocrystal, *e.g.,* a QUANTUM DOT (obtained, for example, from Life Technologies (QuantumDot Corp, Invitrogen Nanocrystal Technologies, Eugene, OR)); see also, U.S. Patent Nos. 6,815,064; 6,682,596; and 6,649,138). Semiconductor nanocrystals are microscopic particles having size-dependent optical and/or electrical properties. When semiconductor nanocrystals are illuminated with a primary energy source, a secondary emission of energy occurs of a frequency that corresponds to the bandgap of the semiconductor material used in the semiconductor nanocrystal. This emission can be detected as colored light of a specific wavelength or fluorescence. Semiconductor nanocrystals with different spectral characteristics are described in *e.g.,* U.S. patent No. 6,602,671. Semiconductor nanocrystals can be coupled to a variety of biological molecules (including dNTPs and/or nucleic acids) or substrates by techniques described in, for example, Bruchez et al., Science 281:2013-2016, 1998; Chan et al., Science 281:2016-2018, 1998; and U.S. Patent No. 6,274,323.

Formation of semiconductor nanocrystals of various compositions are disclosed in, *e.g.,* U.S. Patent Nos. 6,927,069; 6,914,256; 6,855,202; 6,709,929; 6,689,338; 6,500,622; 6,306,736; 6,225,198; 6,207,392; 6,114,038; 6,048,616; 5,990,479; 5,690,807; 5,571,018; 5,505,928; 5,262,357 and in U.S. Patent Publication No. 2003/0165951 as well as PCT Publication No. 99/26299. Separate populations of semiconductor nanocrystals can be produced that are identifiable based on their different spectral characteristics. For example, semiconductor nanocrystals can be produced that emit light of different colors based on their composition, size or size and composition. For example, quantum dots that emit light at different wavelengths based on size (565 nm, 655 nm, 705 nm, or 800 nm emission wavelengths), which are suitable as fluorescent labels in the detection probes disclosed herein are available from Life Technologies (Carlsbad, CA).

Additional labels include, for example, radioisotopes (such as ³H), metal chelates such as DOTA and DPTA chelates of radioactive or paramagnetic metal ions like Gd³⁺, and liposomes.

Detectable labels that can be used with nucleic acid molecules (such as a detection probe) also include enzymes, for example HRP, AP, acid phosphatase, glucose oxidase, β-galactosidase, β-glucuronidase, or β-lactamase. Where the detectable label includes an enzyme, a chromogen, fluorogenic compound, or luminogenic compound can be used in combination with the enzyme to generate a detectable signal (numerous of such compounds are commercially available, for example, from Life Technologies, Carlsbad, CA). Particular examples of chromogenic compounds include diaminobenzidine (DAB), 4-nitrophenylphosphate (pNPP), fast red, fast blue, bromochloroindolyl phosphate (BCIP), nitro blue tetrazolium (NBT), BCIP/NBT, AP Orange, AP blue, tetramethylbenzidine (TMB), 2,2'-azino-di-[3-ethylbenzothiazoline sulphonate] (ABTS), o-dianisidine, 4-chloronaphthol (4-CN), nitrophenyl-β-D-galactopyranoside (ONPG), o-phenylenediamine (OPD), 5-bromo-4-chloro-3-indolyl-β-galactopyranoside (X-Gal), methylumbelliferyl-β-D-galactopyranoside (MU-Gal), p-nitrophenyl-α-D-galactopyranoside (PNP), 5-bromo-4-chloro-3-indolyl-β-D-glucuronide (X-Gluc), 3-amino-9-ethyl carbazol (AEC), fuchsin, iodonitrotetrazolium (INT), tetrazolium blue and tetrazolium violet.

Alternatively, an enzyme can be used in a metallographic detection scheme. Metallographic detection methods include using an enzyme, such as alkaline phosphatase, in combination with a water-soluble metal ion and a redox-inactive substrate of the enzyme. The substrate is converted to a redox-active agent by the enzyme, and the redox-active agent reduces the metal ion, causing it to form a detectable precipitate. (See, for example, U.S. Patent Application Publication No. 2005/0100976, PCT Publication No. 2005/003777 and U.S. Patent Application Publication No. 2004/0265922). Metallographic detection methods also include using an oxido-reductase enzyme (such as horseradish peroxidase) along with a water soluble metal ion, an oxidizing agent and a reducing agent, again to form a detectable precipitate. (See, for example, U.S. Patent No. 6,670,113).

### V. Samples

The samples of use in the disclosed methods include any specimen that includes nucleic acid (such as genomic DNA, cDNA, viral DNA or RNA, rRNA, tRNA, mRNA, miRNA, oligonucleotides, nucleic acid fragments, modified nucleic acids, synthetic nucleic acids, or the like). In some examples, the disclosed methods include obtaining the sample prior to analysis of the sample. In some examples, the disclosed methods include selecting a subject having a tumor, and then in some examples further selecting one or more target nucleic acids including a nucleotide variant to detect based on the subject's tumor, for example, to determine a diagnosis or prognosis for the subject or for selection of one or more therapies. In other examples, the disclosed methods include selecting a subject having, suspected to have, or likely to develop a disorder or condition (such as a heritable genetic disorder, for example, cystic fibrosis, retinitis pigmentosa, muscular dystrophy, or a disease susceptibility gene variant, for example a BRCA1 or BRCA2 mutation), and then in some examples further selecting one or more target nucleic acids including a nucleotide variant to detect based on the subject's known or suspected condition, for example, to determine a diagnosis or prognosis for the subject or for selection of one or more therapies. For example, if the sample is a tumor or cancer sample, the disclosed methods can be used to determine if the cancer is likely to be sensitive or resistant to a therapy, such as those that inhibit EGFR, such as erlotinib, gefitinib, panitumumab and cetuximab (for example by determining if a EGFR mutation is present, which if present, indicates that the cancer is not likely to sensitive to such treatment). Similarly, if the sample is a tumor or cancer sample, the disclosed methods can be used to determine if the cancer is likely to be sensitive or resistant to a therapy, such as those that inhibit mutant BRAF, such as sorafenib, vemurafenib, dabrafenib, or carfilzomib (for example by determining if a BRAF mutation is present, which if present, indicates that the cancer is likely sensitive to such treatment).

Exemplary samples include, without limitation, cells (such as mammalian cells, plant cells, fungal cells, bacterial cells), viruses, cell lysates, blood smears, cytocentrifuge preparations, cytology smears, bodily fluids (*e.g.,* blood, serum, plasma, saliva, sputum, urine, etc.), tissue samples (*e.g*., tissue or tumor biopsies, tissue transplants, xenographs, or fixed and/or embedded tissue samples), fine-needle aspirates, tissue sections (*e.g*., cryostat tissue sections and/or paraffin-embedded tissue sections), buccal swabs, cervical swabs, and/or environmental samples (such as food, water, soil, air filter, or water filter samples). In other examples, the sample includes isolated nucleic acid (such as genomic DNA, cDNA, RNA, mRNA) from a subject, for example nucleic acid isolated from cells, cell lysates, blood smears, cytology smears, bodily fluids, tissue biopsies, fine-needle aspirates, and/or tissue sections from a subject.

Methods of obtaining a sample from a subject are known in the art. For example, methods of obtaining bodily fluid, tissue, or cell samples are routine. Methods of isolating nucleic acids from samples are also routine. Exemplary samples may be obtained from normal cells or tissues, or from neoplastic cells or tissues. Neoplasia is a biological condition in which one or more cells have undergone characteristic anaplasia with loss of differentiation, increased rate of growth, invasion of surrounding tissue, and which cells may be capable of metastasis. In particular examples, a biological sample includes a tumor sample, such as a sample containing neoplastic cells. Exemplary neoplastic cells or tissues may be included in or isolated from solid tumors, including lung cancer (*e.g*., non-small cell lung cancer, such as lung squamous cell carcinoma), breast carcinomas (*e.g*. lobular and duct carcinomas), adrenocortical cancer, ameloblastoma, ampullary cancer, bladder cancer, bone cancer, cervical cancer, cholangioma, colorectal cancer, endometrial cancer, esophageal cancer, gastric cancer, glioma, granular call tumor, head and neck cancer, hepatocellular cancer, hydatiform mole, lymphoma, melanoma, mesothelioma, myeloma, neuroblastoma, oral cancer, osteochondroma, osteosarcoma, ovarian cancer, pancreatic cancer, pilomatricoma, prostate cancer, renal cell cancer, salivary gland tumor, soft tissue tumors, Spitz nevus, squamous cell cancer, teratoid cancer, and thyroid cancer. Exemplary neoplastic cells may also be included in or isolated from hematological cancers including leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, and myelodysplasia.

For example, a sample from a tumor that contains cellular material can be obtained by surgical excision of all or part of the tumor, by collecting a fine needle aspirate from the tumor, as well as other methods known in the art. In some examples, a tissue or cell sample is applied to a substrate and analyzed to determine presence or absence of one or more nucleotide variants. A solid support useful in a disclosed method need only bear the biological sample and, optionally, but advantageously, permit the convenient detection of components (*e.g*., proteins and/or nucleic acid sequences) in the sample. Exemplary supports include microscope slides (*e.g.,* glass microscope slides or plastic microscope slides), coverslips (*e.g.,* glass coverslips or plastic coverslips), tissue culture dishes, multi-well plates, membranes (*e.g*., nitrocellulose or polyvinylidene fluoride (PVDF)) or BIACORE™ chips.

The samples described herein can be prepared using any method now known or hereafter developed in the art. In some examples, cells in the sample are lysed or permeabilized in an aqueous solution (for example using a lysis buffer). The aqueous solution or lysis buffer includes detergent (such as sodium dodecyl sulfate) and one or more chaotropic agents (such as formamide, guanidinium HCl, guanidinium isothiocyanate, or urea). The solution may also contain a buffer (for example SSC). In some examples, the lysis buffer includes about 15% to 25% formamide (v/v) about 0.01% to 0.1% SDS, and about 0.5-6X SSC (for example, about 3X SSC). The buffer may optionally include tRNA (for example, about 0.001 to about 2.0 mg/ml) or a ribonuclease inhibitor. The lysis buffer may also include a pH indicator, such as Phenol Red, EDTA (for example, about 1 mM or less), and/or proteinase K (for example about 0.1 to 2 mg/ml). In a particular example, the lysis buffer includes 20% formamide, 3X SSC (79.5%), 0.05% SDS, 1 µg/ml tRNA, and 1 mg/ml Phenol Red. Cells are incubated in the aqueous solution for a sufficient period of time (such as about 1 minute to about 60 minutes, for example about 5 minutes to about 20 minutes, or about 10 minutes) and at a sufficient temperature (such as about 22°C to about 115°C, for example, about 37°C to about 105°C, or about 90°C to about 100°C) to lyse or permeabilize the cell. In some examples, lysis is performed at about 95°C. In some examples, the lysis step includes incubating the sample at about 95°C for about 5-15 minutes to denature RNA in the sample, but not genomic DNA. In other examples, the lysis step includes incubating the sample at about 105°C for about 5-15 minutes to denature both RNA and genomic DNA in the sample.

In some examples, the crude cell lysis is used directly without further purification ("lysis only" sample preparation). The cells may be lysed in the presence or absence of one or more of the disclosed detection probes. If the cells are lysed in the absence of detection probe(s), the one or more detection probes can be subsequently added to the crude lysate. In other examples, nucleic acids are isolated from the cell lysate prior to contacting with one or more of the disclosed detection probes. In some examples, isolated nucleic acids (such as RND or DNA) are added to lysis buffer, and then the one or more detection probes are subsequently added. In some examples, the methods do not include nucleic amplification (for example, nucleic acid amplification is not performed prior to contacting the sample with the detection probe(s), bifunctional oligonucleotide(s), and/or surface(s)). However, in some examples, the disclosed methods can include nucleic acid purification, nucleic acid amplification, and/or pre-processing of the sample (for example, instead of or in addition to cell lysis).

In other examples, tissue samples are prepared by fixing and embedding the tissue in a medium or include a cell suspension prepared as a monolayer on a solid support (such as a glass slide), for example by smearing or centrifuging cells onto the solid support. In other examples, a cell pellet is prepared by sedimenting a population of cells (such as cells obtained from a tissue sample or cultured cells). The cell pellet can further be fixed and embedded in an embedding medium for analysis. In further examples, fresh frozen (for example, unfixed) tissue or tissue sections may be used in the methods disclosed herein. In particular examples, FFPE tissue sections are used in the disclosed methods.

The disclosure is further illustrated by the following non-limiting Examples.

### EXAMPLES

### Example 1

### Detection of BRAF V600 Variants in In vitro Transcripts

This example describes detection of BRAF V600 wild type or variants in *in vitro* transcripts (IVTs) utilizing a direct detection/ligation assay.

Synthetic IVT mRNAs for each wild type or variant sequence were synthesized. The sequences of various V600 variants are shown in Table 2. Detection probe and target-specific portions of bifunctional oligonucleotides for BRAF V600 wild type and variants are shown in Table 3. The detection probe was 5' phosphorylated and biotin-labeled through incorporation of a biotin-labeled thymidine nucleotide.

**Table 2. BRAF V600 mutations are very similar to each other**

| **Genotype** | **Sequence (5'-3')** | **SEQ ID NO:** |
|---|---|---|
| V600wt | GGTGATTTTGGTCTAGCTACAGTGAAATCTCGATGGA | 1 |
| V600E | GGTGATTTTGGTCTAGCTACAG***A***GAAATCTCGATGGA | 2 |
| V600K | GGTGATTTTGGTCTAGCTACA***AA***GAAATCTCGATGGA | 3 |
| V600E2 | GGTGATTTTGGTCTAGCTACAG***AA***AAATCTCGATGGA | 4 |
| V600D | GGTGATTTTGGTCTAGCTACAG***AT***AAATCTCGATGGA | 5 |

**Table 3. BRAF V600 detection probe and target-specific portion of bifunctional oligonucleotides**

| **Oligo** | **Sequence (5'-3')** | **SEQ ID NO:** |
|---|---|---|
| V600 Probe | TGTAGCTAGACCAAAATCACCTATTTTTACTGTGAGG | 6 |
| V600 wt bifunctional oligo (target-specific portion) | CTGATGGGACCCACTCCATCGAGATTTCA | 7 |
| V600E bifunctional oligo (target-specific portion) | CTGATGGGACCCACTCCATCGAGATTTCT | 8 |
| V600K bifunctional oligo (target-specific portion) | CTGATGGGACCCACTCCATCGAGATTTCTT | 9 |
| V600E2 bifunctional oligo (target-specific portion) | CTGATGGGACCCACTCCATCGAGATTTTTC | 10 |
| V600D bifunctional oligo (target-specific portion) | CTGATGGGACCCACTCCATCGAGATTTATC | 11 |

IVT was diluted in lysis buffer to a concentration to provide 50 fM total RNA per well final concentration. An equal volume of high salt solution (2:1:1 of S1 nuclease buffer: S1 stop solution: neutralization buffer) was added and 25 µl/well was added to an ArrayPlate (which includes immobilized addressable anchors). 70 µl of denaturation oil and 5 µl of 6X detection probes and bifunctional oligonucleotides were added to each well (final concentration of detection probe was 167 pM and final concentration of each bifunctional oligonucleotide was 30 pM). The plate was incubated at 80°C for 15 minutes, then at 37°C overnight (16-24 hours) with shaking (300 rpm). The plate was washed and 50 µl/well of modified T4 ligation solution was added (50 mM Tris pH 7.6, 1 mM MgCl₂, 10 mM DTT, 2 µM ATP, 0.5% TWEEN®-20) with 4 U/µl T4 DNA ligase (New England Biolabs, catalog number M0202L). The plate was incubated at 37°C for 2 hours with shaking (300 rpm), then 50 µl/well of 2X RNase H solution (100 mM Tris pH 8.0, 150 mM KCl, 6 mM MgCl₂, 20 mM DTT) was added (without removal of the ligation solution) with 0.025 U/µl RNase H (New England Biolabs, catalog number M0297L) and incubated at 37°C for 1 hour with shaking at 300 rpm. Next, 50 µl/well of proteinase K solution at 0.2 mg/ml in 3X Proteinase K Buffer (150 mM Tris pH 8.0, 450 mM NaCl, 0.3% SDS) (Ambion, catalog number M2546) was added (without removal of RNase H solution) and incubated at 50°C for 1 hour with shaking (300 rpm).

The plate was washed and detection was performed with streptavidin Poly HRP-80 conjugate. Briefly, 50 µl/well of pre-block solution (Universal Casein Diluent/Blocker, Fitzgerald Industries International, Cat No 85R-108) was added and the plate was incubated at room temperature for 20 minutes with shaking (300 rpm). Without removing the pre-block solution, 50 µl/well of Streptavidin Poly-HRP80 conjugate (Fitzgerald Industries, catalog number 65R-S105PHRP) at 1:1250 in Universal Casein Diluent was added and the plate was incubated at room temperature with shaking (300 rpm) for 20 minutes. The plate was washed twice, and 50 µl/well of luminescent substrate solution comprised of equal volumes of Lumigen TMA-3 Substrate A and B (Cat No. TM3-1000, Lumigen, Inc.) and 100 µl/well of imaging oil were added. Image capture was performed on OMIX imager.

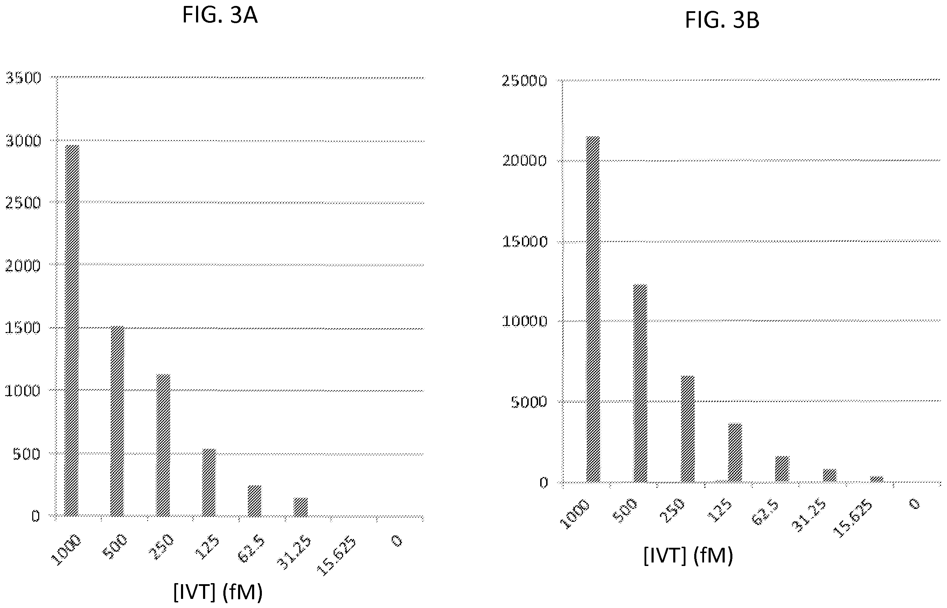

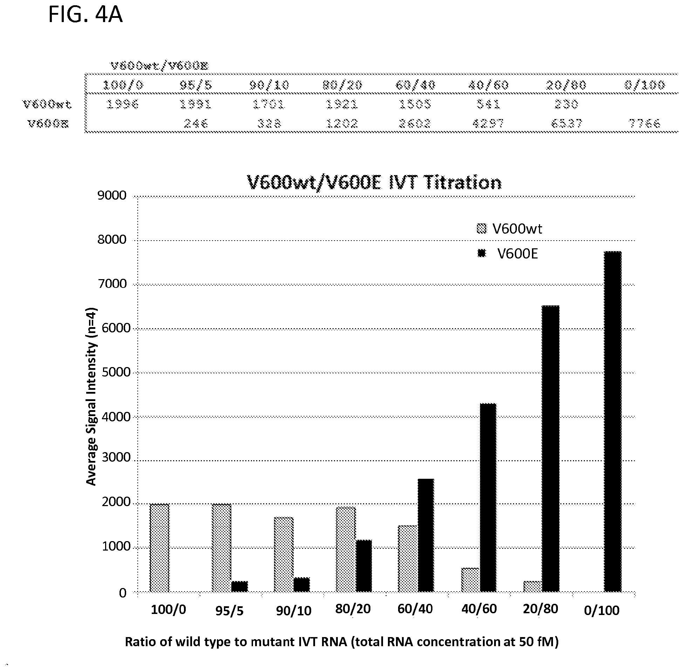

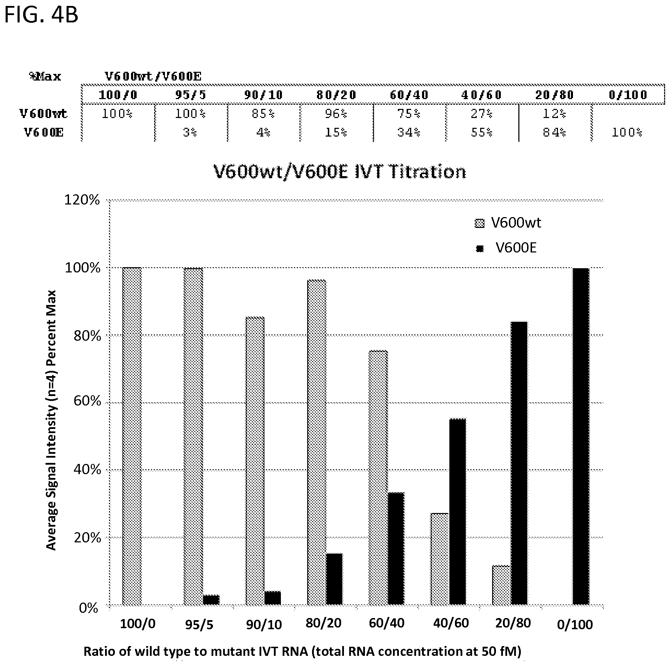

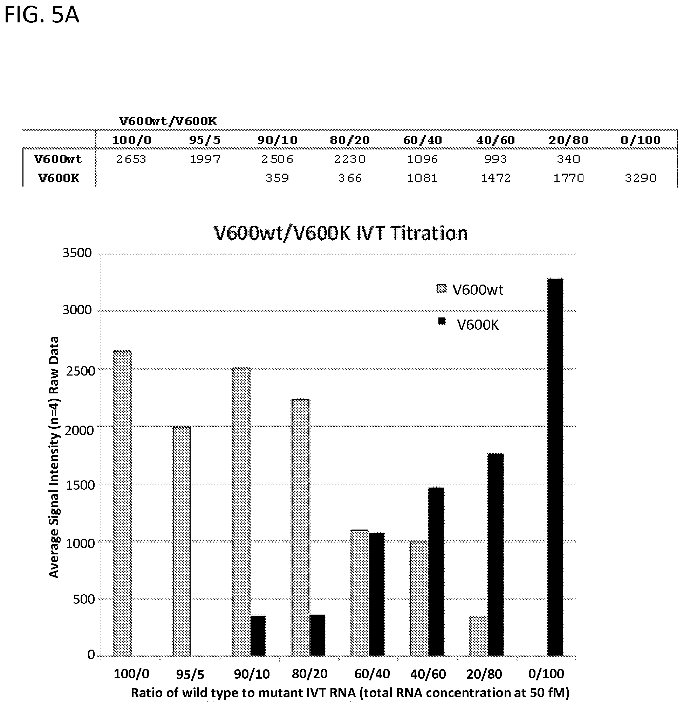

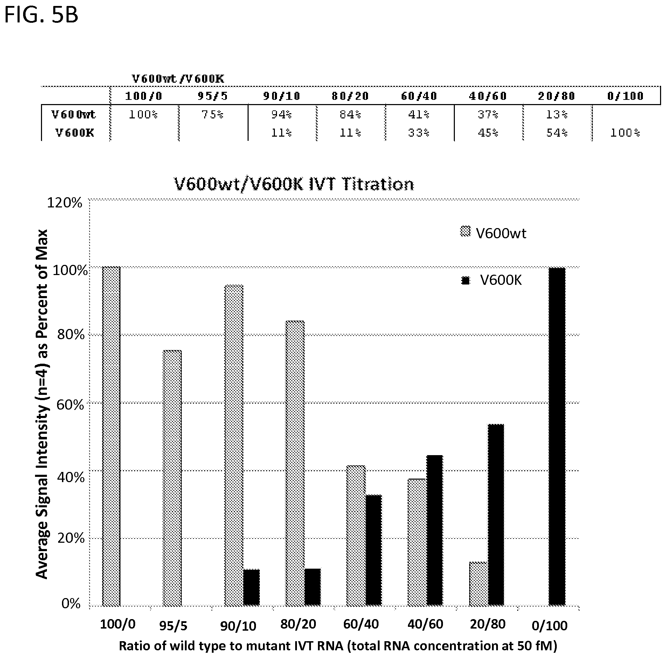

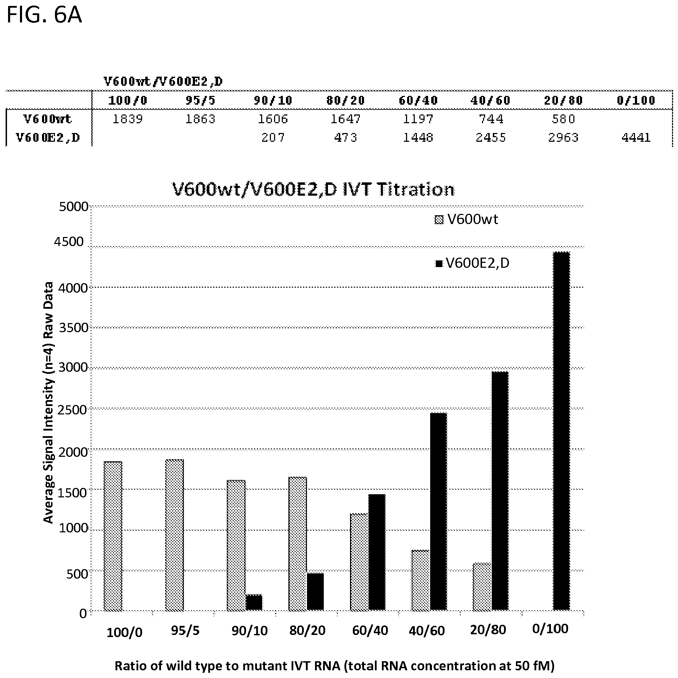

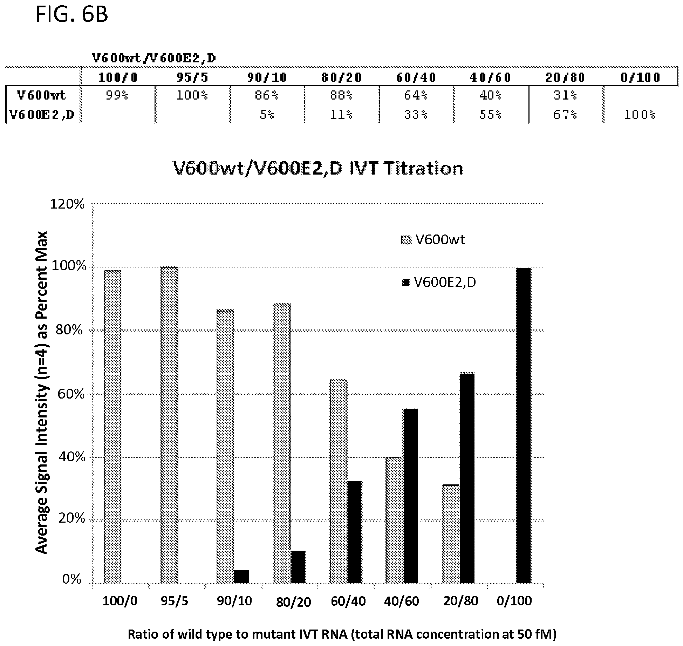

Detection of varying amounts of BRAF V600 wild type and variant IVTs were assayed. As shown in FIGS. 3A and 3B, the signal from the wild type detection probe (FIG. 3A) was substantially less than from the V600E detection probe (FIG. 3B); however, the specificity was excellent. A mixture of V600 wild type and variant IVTs was also used in the assay. V600E IVT could be detected even when present at only 5% of the transcript amount (FIGS. 4A and 4B). V600K or V600E2/D IVTs could be detected when present at only 10% of the transcript amount (FIGS. 5A and 5B and 6A and 6B).

### Example 2

### Detection of BRAF V600E Variant in Cell Lines

This example describes detection of BRAF V600 wild type or V600E variant in cell lysates.

Presence of BRAF V600E was assayed in cell lysates from melanoma cell lines of known BRAF genotype using the protocol described in Example 1, except that the starting sample was cell lysate prepared from 6000 cells or 50,000 cells, as indicated. The cell lines and their genotypes are shown in Table 4. All cell lines were primary malignant melanoma cell lines.

**Table 4. Melanoma cell lines with known BRAF V600 variants**

| **Cell Line** | **BRAF Genotype** | **Homozygous/Heterozygous** |
|---|---|---|
| SK-MEL-2 | V600wt | homozygous |
| SK-MEL-1 | V600E | heterozygous |
| A375 | V600E | homozygous |
| G-361 | V600E | heterozygous |

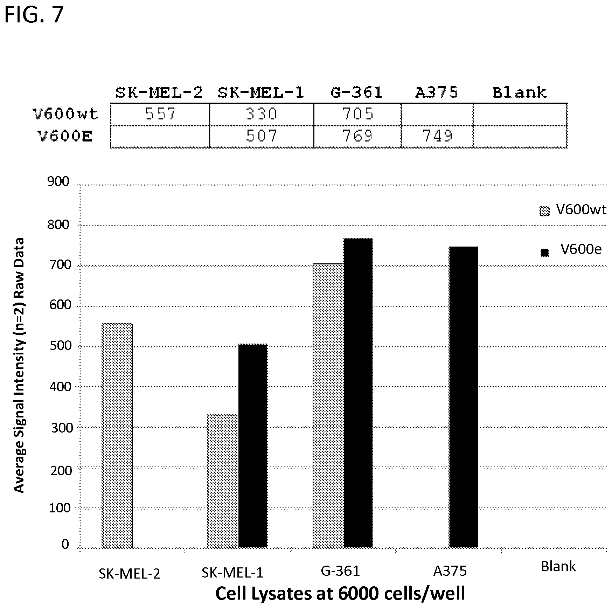

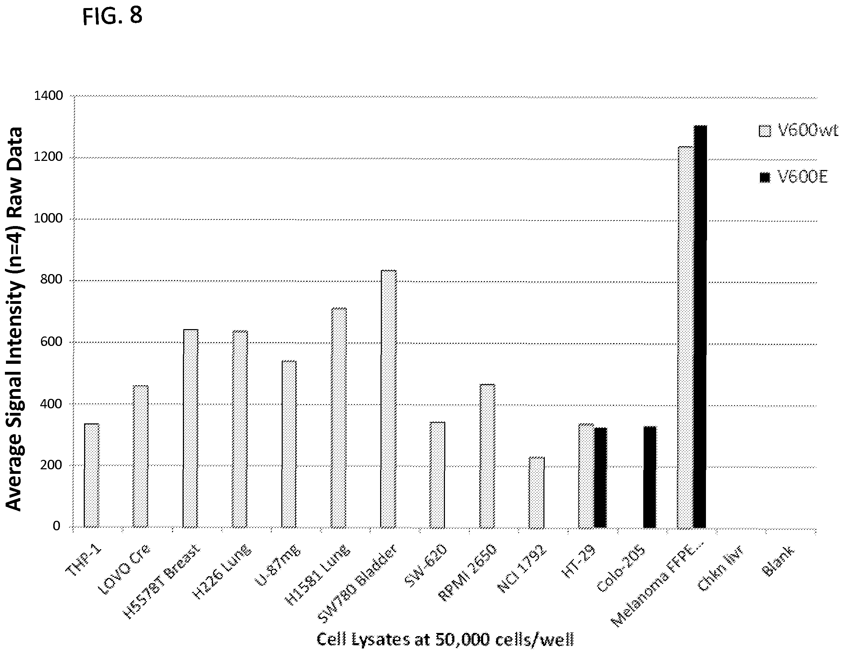

Results of the assay for each cell line are shown in FIG. 7. The genotype of each cell line was correctly identified by the assay. Additional cell lines were tested (FIG. 8). All tested cell lines were wild type at BRAF V600, except for HT-29 and Colo-205 colon cancer cell lines. HT-29 was heterozygous for V600E and Colo-205 was homozygous for V600E. A melanoma FFPE sample tested in the same assay was also heterozygous for V600E.

### Example 3

### Detection of BRAF V600E Variant in Melanoma Samples

This example describes detection of BRAF V600 wild type or V600E variant in melanoma samples.

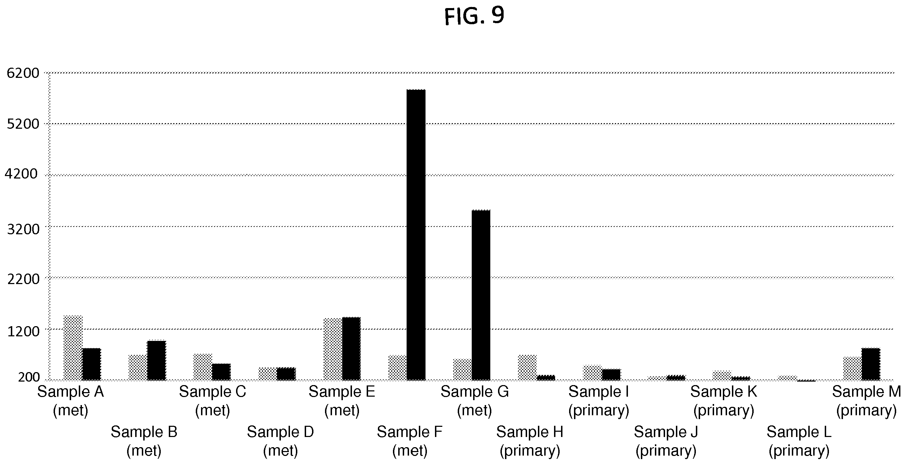

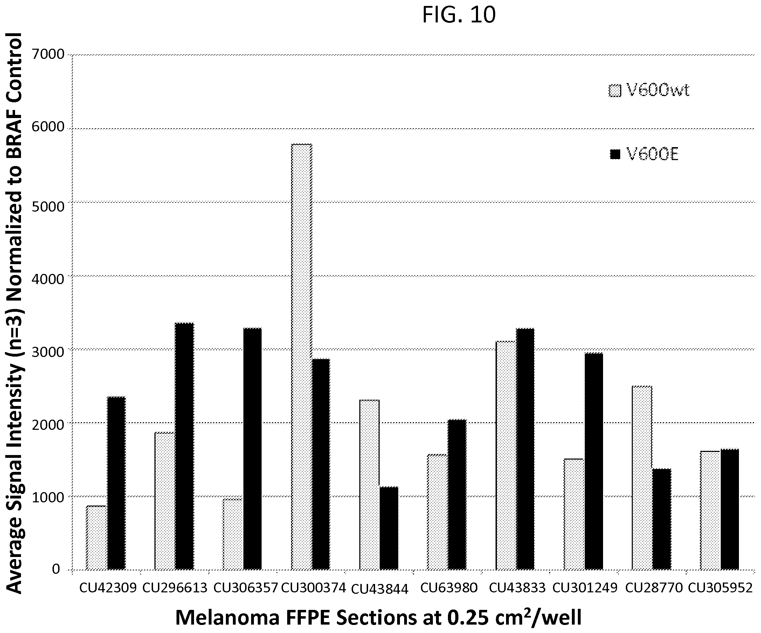

FFPE samples were scraped off the slide and solubilized in Lysis Buffer at 0.5 cm² per well (25 µl) then diluted to a final of 0.25 cm² per well by the addition of high salt buffer and were assayed for BRAF V600/V600E as described in Example 1. A series of FFPE samples of primary or metastatic melanoma were tested for present of BRAF V600E mutation (FIG. 9). An additional series of 10 FFPE melanoma samples known to have the BRAF V600E were also tested (Cureline, Inc., South San Francisco, CA). The BRAF V600E mutation was detected in all of the samples (FIG. 10).

### Example 4

### Optimizing Assay Sensitivity

This example describes optimization of assay sensitivity for detection of BRAF V600E variant.

Signal amplification with streptavidin poly-HRP conjugates was investigated to increase assay sensitivity. Detection with streptavidin poly-HRP 20 (Fitzgerald Industries International, Acton, MA) increased signal intensity by 11-fold over standard avidin peroxidase detection with a signal to noise ratio of 11.3. Use of streptavidin poly-HRP 80 (Fitzgerald Industries International) increased signal intensity by 36-fold over standard avidin peroxidase detection with a signal to noise ratio of 27.6 at a 1:1000 dilution. In addition to the increase in signal intensity resulting from use of poly-HRP 80, the background increased from essentially 0 to about 200. It is believed that some of the background signal is the result of trace contamination of the anchors or bifunctional oligonucleotides with biotin. More rigorous purification of the anchors and bifunctional oligonucleotides to remove trace levels of biotin may reduce this background.

In view of the many possible embodiments to which the principles of the disclosure may be applied, it should be recognized that the illustrated embodiments are only examples and should not be taken as limiting the scope of the invention. Rather, the scope of the invention is defined by the following claims.

## Claims

1. A method of detecting the presence of a target nucleic acid molecule in a sample, comprising:
a) contacting the sample with:
i) at least one oligonucleotide detection probe comprising a detectable label,
wherein the detection probe specifically binds to a first target sequence of the target nucleic acid molecule, optionally wherein the detection probe comprises 15 to 50 nucleotides or 20 to 30 nucleotides;
ii) at least one bifunctional oligonucleotide that comprises:
A) a target-specific portion, which specifically binds to a second target sequence of the target nucleic acid molecule that is contiguous to the first target sequence; and
B) an anchor-specific portion, which specifically binds to an anchor; and
iii) at least one surface comprising the anchor, which is immobilized on the surface; under conditions sufficient for:
I) the detection probe to specifically bind to the first target sequence;
II) the target-specific portion of the bifunctional oligonucleotide to specifically bind to the second target sequence; and
III) the anchor to specifically bind to the anchor-specific portion of the bifunctional oligonucleotide, such that the target nucleic acid, the detection probe, and the bifunctional oligonucleotide are directly or indirectly bound to the surface via the anchor, thereby producing a tethered target nucleic acid, a tethered detection probe, and a tethered bifunctional oligonucleotide,
wherein one or more of steps i), ii), and iii) may occur contemporaneously or sequentially;
b) adding a ligase to the tethered detection probe and the tethered bifunctional oligonucleotide, thereby producing a ligated detection probe and bifunctional oligonucleotide;
c) adding a reagent to specifically remove substantially all tethered target nucleic acid molecule, wherein the reagent has substantially no specific activity to remove the ligated detection probe and bifunctional oligonucleotide;
d) removing substantially all unligated detection probe; and
e) detecting on the at least one surface the presence of the detectable label at the position of the anchor, thereby detecting presence of the target nucleic acid molecule in the sample.

2. The method of claim 1, wherein the sample is suspended in an aqueous solution and the sample is:
(a) contacted with the detection probe, the bifunctional oligonucleotide, and the at least one surface comprising the anchor contemporaneously; or
(b) contacted with:
i) the detection probe; and
ii) the bifunctional oligonucleotide and the at least one surface comprising the anchor,
wherein steps i) and ii) are performed sequentially.

3. The method of claim 1 or claim 2, wherein the target nucleic acid molecule comprises RNA, the detection probe and the bifunctional oligonucleotide comprise DNA, and the reagent comprises a RNA-specific nuclease.

4. The method of any one of claims 1 to 3, wherein the detectable label comprises a hapten, a fluorescent molecule, an enzyme, or a radioisotope, optionally wherein the label comprises biotin.

5. The method of any one of claims 1 to 4, wherein the detection probe is end-labeled.

6. The method of any one of claims 1 to 5, wherein the conditions in step (a) comprise incubating the sample, the detection probe, the bifunctional oligonucleotide, and the at least one surface comprising the anchor at about 37-50°C for about 4-48 hours.

7. The method of any one of claims 1 to 6, wherein the bifunctional oligonucleotide comprises 18 to 75 nucleotides, optionally wherein the bifunctional oligonucleotide further comprises a spacer between the target-specific portion and anchor-specific portion, optionally wherein the spacer comprises 6 to 36 carbon atoms or 5 to 50 nucleotides.

8. The method of any one of claims 1 to 7, wherein the anchor comprises about 15 to 150 nucleotides, optionally wherein the anchor is an addressable anchor.

9. The method of any one of claims 1 to 8, wherein the at least one surface comprising the anchor comprises one or more membranes, one or more plates, one or more wells, one or more tubes, one or more beads, or one or more microfluidic channels.

10. The method of any one of claims 1 to 9, wherein the sample comprises tissue, fixed tissue, a tumor biopsy, cells, blood, a bodily fluid, or isolated nucleic acid molecules.

11. The method of claim any one of claims 1 to 10, wherein the ligase comprises T4 DNA ligase and/or wherein the reagent in step (c) comprises ribonuclease H, ribonuclease A, and/or ribonuclease T1.

12. The method of any one of claims 1 to 11, wherein the detection probe or the bifunctional oligonucleotide comprises a 5' phosphate moiety.

13. The method of any one of claims 1 to 12, wherein a position of at least one nucleotide in the first target sequence or the second target sequence comprises a variant nucleotide or a wild-type nucleotide, wherein the position is a variant nucleotide position (VNP) and the VNP is located no more than three nucleotides from the junction of the contiguous first target sequence and second target sequence, optionally wherein the VNP is adjacent to the junction of the first target sequence and the second target sequence.

14. The method of claim 13, wherein step (a)(ii) comprises contacting the sample with at least two bifunctional oligonucleotides, one of which specifically binds to the wild-type nucleotide at the VNP of the second target sequence, and the other of which specifically binds to the variant nucleotide at the VNP of the second target sequence, optionally wherein the anchor-specific portions of the at least two bifunctional oligonucleotides specifically bind to different anchors immobilized on the surface.

## Patentansprüche

1. Nachweisverfahren für die Anwesenheit eines Zielnukleinsäuremoleküls in einer Probe, das Folgendes umfasst:
a) Kontaktieren der Probe mit:
i) mindestens einer Oligonukleotidnachweissonde, die eine nachweisbare Markierung umfasst, wobei die Nachweissonde eine erste Zielsequenz des Zielnukleinsäuremoleküls spezifisch bindet, optional wobei die Nachweissonde 15 bis 50 Nukleotide oder 20 bis 30 Nukleotide umfasst;
ii) mindestens einem bifunktionellen Oligonukleotid, der Folgendes umfasst:
A) einen zielspezifischen Abschnitt, der eine zweite Zielsequenz des Zielnukleinsäuremoleküls, die mit der ersten Zielsequenz zusammenhängt, spezifisch bindet; und
B) einen ankerspezifischen Abschnitt, der einen Anker spezifisch bindet; und
iii) mindestens einer Fläche, die den Anker umfasst, der auf der Fläche immobilisiert ist; unter Bedingungen, die ausreichend sind für:
I) die Nachweissonde, um die erste Zielsequenz spezifisch zu binden;
II) den zielspezifischen Abschnitt des bifunktionellen Oligonukleotids, um die zweite Zielsequenz spezifisch zu binden; und
III) den Anker, um den ankerspezifischen Abschnitt des bifunktionellen Oligonukleotids spezifisch zu binden, sodass die Zielnukleinsäure, die Nachweissonde und der bifunktionelle Oligonukleotid direkt oder indirekt über den Anker an die Fläche gebunden sind, wodurch eine gebundene Zielnukleinsäure, eine gebundene Nachweissonde und ein gebundener bifunktioneller Oligonukleotid produziert werden,
wobei einer oder mehrere der Schritte i), ii) und iii) gleichzeitig oder nacheinander auftreten können;
b) Hinzufügen einer Ligase zu der gebundenen Nachweissonde und dem gebundenen bifunktionellen Oligonukleotid, wodurch eine ligierte Nachweissonde und ein ligierter bifunktioneller Oligonukleotid produziert werden;
c) Hinzufügen eines Reagenzes, um im Wesentlichen alle gebundenen Zielnukleinsäuremoleküle spezifisch zu entfernen, wobei das Reagenz im Wesentlichen keine spezifische Aktivität aufweist, um die ligierte Nachweissonde und den ligierten bifunktionellen Oligonukleotid zu entfernen;
d) Entfernen von im Wesentlichen allem von der unligierten Nachweissonde; und
e) Nachweisen der Anwesenheit der nachweisbaren Markierung auf der mindestens einen Fläche bei einer Position des Ankers, wodurch eine Anwesenheit des Zielnukleinsäuremoleküls in der Probe nachgewiesen wird.

2. Verfahren nach Anspruch 1, wobei die Probe in einer wässrigen Lösung suspendiert wird, und wobei die Probe Folgendes wird:
(a) mit der Nachweissonde, dem bifunktionellen Oligonukleotid und der mindestens einen Fläche, die den Anker umfasst, gleichzeitig kontaktiert; oder
(b) kontaktiert mit:
i) der Nachweissonde; und
ii) dem bifunktionellen Oligonukleotid und der mindestens einen Fläche, die den Anker umfasst,
wobei die Schritte i) und ii) nacheinander durchgeführt werden.

3. Verfahren nach Anspruch 1 oder Anspruch 2, wobei das Zielnukleinsäuremolekül RNS umfasst, wobei die Nachweissonde und der bifunktionelle Oligonukleotid DNS umfassen, und wobei das Reagenz eine RNS-spezifische Nuklease umfasst.

4. Verfahren nach einem der Ansprüche 1 bis 3, wobei die nachweisbare Markierung ein Hapten, ein fluoreszierendes Molekül, ein Enzym oder ein Radioisotop umfasst, optional wobei die Markierung Biotin umfasst.

5. Verfahren nach einem der vorhergehenden Ansprüche 1 bis 4, wobei die Nachweissonde endmarkiert ist.

6. Verfahren nach einem der Ansprüche 1 bis 5, wobei die Bedingungen in Schritt (a) ein Inkubieren der Probe, der Nachweissonde, des bifunktionellen Oligonukleotids und der mindestens einen Fläche, die den Anker umfasst, bei etwa 37-50 °C für etwa 4-48 Stunden umfassen.

7. Verfahren nach einem der Ansprüche 1 bis 6, wobei der bifunktionelle Oligonukleotid 18 bis 75 Nukleotide umfasst, optional wobei der bifunktionelle Oligonukleotid ferner einen Abstandhalter zwischen dem zielspezifischen Abschnitt und dem ankerspezifischen Abschnitt umfasst, optional wobei der Abstandhalter 6 bis 36 Kohlenstoffatome oder 5 bis 50 Nukleotide umfasst.

8. Verfahren nach einem der Ansprüche 1 bis 7, wobei der Anker etwa 15 bis 150 Nukleotide umfasst, optional wobei der Anker ein adressierbarer Anker ist.

9. Verfahren nach einem der Ansprüche 1 bis 8, wobei die mindestens eine Fläche, die den Anker umfasst, eine oder mehrere Membranen, eine oder mehrere Platten, eine oder mehrere Vertiefungen, eine oder mehrere Röhren, eine oder mehrere Kügelchen oder einen oder mehrere Mikrofluidkanäle umfasst.

10. Verfahren nach einem der Ansprüche 1 bis 9, wobei die Probe Gewebe, fixiertes Gewebe, eine Tumorbiopsieprobe, Zellen, Blut, eine Körperflüssigkeit oder isolierte Nukleinsäuremoleküle umfasst.

11. Verfahren nach Anspruch einem der Ansprüche 1 bis 10, wobei die Ligase eine T4-DNS-Ligase umfasst und/oder wobei das Reagenz in Schritt (c) Ribonuklease H, Ribonuklease A und/oder Ribonuklease T1 umfasst.

12. Verfahren nach einem der Ansprüche 1 bis 11, wobei die Nachweissonde und der bifunktionelle Oligonukleotid einen 5'-Phospahtrest umfassen.

13. Verfahren nach einem der Ansprüche 1 bis 12, wobei eine Position von mindestens einem Nukleotid in der ersten Zielsequenz oder der zweiten Zielsequenz einen varianten Nukleotid oder einen Wildtypnukleotid umfasst, wobei die Position eine variante Nukleotidposition (VNP) ist, und wobei die VNP sich nicht mehr als drei Nukleotide von der Verbindung zwischen der zusammenhängen ersten Zielsequenz und der zweiten Zielsequenz befindet, optional wobei die VNP der Verbindung der ersten Zielsequenz und der zweiten Zielsequenz benachbart ist.

14. Verfahren nach Anspruch 13, wobei der Schritt (a) (ii) ein Kontaktieren der Probe mit mindestens zwei bifunktionellen Oligonukleotiden umfasst, wobei einer davon den Wildtypnukleotid an der VNP der zweiten Zielsequenz bindet und der andere davon den varianten Nukleotid an der VNP der zweiten Zielsequenz bindet, optional wobei der ankerspezifische Abschnitt der mindestens zwei bifunktionellen Oligonukleotide verschiedene Anker, die auf der Fläche immobilisiert sind, spezifisch bindet.

## Revendications

1. Procédé de détection de la présence d'une molécule d'acide nucléique cible dans un échantillon comprenant :
a) la mise en contact de l'échantillon avec :
i) au moins une sonde de détection d'oligonucléotide comprenant un marqueur détectable, dans lequel la sonde de détection se lie spécifiquement à une première séquence cible de la molécule d'acide nucléique cible, éventuellement dans lequel la sonde de détection comprend entre 15 et 50 nucléotides ou entre 20 et 30 nucléotides ;
ii) au moins un oligonucléotide bifonctionnel qui comprend :
A) une partie spécifique à la cible, qui se lie spécifiquement à une seconde séquence cible de la molécule d'acide nucléique cible qui est contiguë à la première séquence cible ; et
B) une partie spécifique à l'ancrage, qui se lie spécifiquement à un ancrage ; et
iii) au moins une surface comprenant l'ancrage, qui est immobilisé sur la surface ; dans des conditions suffisantes pour que :
I) la sonde de détection se lie spécifiquement à la première séquence cible ;
II) la partie spécifique à la cible de l'oligonucléotide bifonctionnel se lie spécifiquement à la seconde séquence cible ; et
III) l'ancrage se lie spécifiquement à la partie spécifique à l'ancrage de l'oligonucléotide bifonctionnel, de sorte que l'acide nucléique cible, la sonde de détection et l'oligonucléotide bifonctionnel sont directement ou indirectement liés à la surface par l'intermédiaire de l'ancrage, produisant ainsi un acide nucléique cible fixé, une sonde de détection fixée et un oligonucléotide bifonctionnel fixé,
dans lequel l'une ou plusieurs des étapes i), ii) et iii) peuvent se produire de manière simultanée ou séquentielle ;
b) l'ajout d'une ligase à la sonde de détection fixée et à l'oligonucléotide bifonctionnel fixé, produisant ainsi une sonde de détection et un oligonucléotide bifonctionnel ligaturés ;
c) l'ajout d'un réactif pour éliminer spécifiquement sensiblement l'ensemble de la molécule d'acide nucléique cible fixée, dans lequel le réactif n'a sensiblement aucune activité spécifique pour éliminer la sonde de détection et l'oligonucléotide bifonctionnel ligaturés ;
d) l'élimination de sensiblement l'ensemble de la sonde de détection non ligaturée ; et
e) la détection sur l'au moins une surface de la présence du marqueur détectable au niveau de la position de l'ancrage, détectant ainsi la présence de la molécule d'acide nucléique cible dans l'échantillon.

2. Procédé selon la revendication 1, dans lequel l'échantillon est mis en suspension dans une solution aqueuse et l'échantillon est :
(a) mis en contact avec la sonde de détection, l'oligonucléotide bifonctionnel et l'au moins une surface comprenant l'ancrage de manière simultanée ; ou
(b) mis en contact avec :
i) la sonde de détection ; et
ii) l'oligonucléotide bifonctionnel et l'au moins une surface comprenant l'ancrage,
dans lequel les étapes i) et ii) sont réalisées de manière séquentielle.

3. Procédé selon la revendication 1 ou la revendication 2, dans lequel la molécule d'acide nucléique cible comprend de l'ARN, la sonde de détection et l'oligonucléotide bifonctionnel comprennent de l'ADN et le réactif comprend une nucléase spécifique à l'ARN.

4. Procédé selon l'une quelconque des revendications 1 à 3, dans lequel le marqueur détectable comprend un haptène, une molécule fluorescente, une enzyme ou un radioisotope, éventuellement dans lequel le marqueur comprend de la biotine.

5. Procédé selon l'une quelconque des revendications 1 à 4, dans lequel la sonde de détection est marquée aux extrémités.

6. Procédé selon l'une quelconque des revendications 1 à 5, dans lequel les conditions de l'étape (a) comprennent l'incubation de l'échantillon, de la sonde de détection, de l'oligonucléotide bifonctionnel et de l'au moins une surface comprenant l'ancrage à environ 37 à 50 °C pendant environ 4 à 48 heures.

7. Procédé selon l'une quelconque des revendications 1 à 6, dans lequel l'oligonucléotide bifonctionnel comprend entre 18 et 75 nucléotides, éventuellement dans lequel l'oligonucléotide bifonctionnel comprend en outre un espaceur entre la partie spécifique à la cible et la partie spécifique à l'ancrage, éventuellement dans lequel l'espaceur comprend entre 6 et 36 atomes de carbone ou entre 5 et 50 nucléotides.

8. Procédé selon l'une quelconque des revendications 1 à 7, dans lequel l'ancrage comprend entre environ 15 et 150 nucléotides, éventuellement dans lequel l'ancrage est un ancrage pouvant être adressé.

9. Procédé selon l'une quelconque des revendications 1 à 8, dans lequel l'au moins une surface comprenant l'ancrage comprend une ou plusieurs membranes, une ou plusieurs plaques, un ou plusieurs puits, un ou plusieurs tubes, une ou plusieurs billes, ou un ou plusieurs canaux microfluidiques.

10. Procédé selon l'une quelconque des revendications 1 à 9, dans lequel l'échantillon comprend un tissu, un tissu fixé, une biopsie de tumeur, des cellules, du sang, un fluide corporel ou des molécules d'acide nucléique isolées.

11. Procédé selon l'une quelconque des revendications 1 à 10, dans lequel la ligase comprend une ligase d'ADN T4 et/ou dans lequel le réactif de l'étape (c) comprend une ribonucléase H, une ribonucléase A et/ou une ribonucléase T1.

12. Procédé selon l'une quelconque des revendications 1 à 11, dans lequel la sonde de détection ou l'oligonucléotide bifonctionnel comprend une fraction phosphate 5'.

13. Procédé selon l'une quelconque des revendications 1 à 12, dans lequel la position d'au moins un nucléotide dans la première séquence cible ou dans la seconde séquence cible comprend un nucléotide variant ou un nucléotide de type sauvage, dans lequel la position est une position de nucléotide variant (VNP) et la VNP est située au plus à trois nucléotides de la jonction de la première séquence cible et de la seconde séquence cible contiguës, éventuellement dans lequel la VNP est adjacente à la jonction de la première séquence cible et de la seconde séquence cible.

14. Procédé selon la revendication 13, dans lequel l'étape (a) (ii) comprend la mise en contact de l'échantillon avec au moins deux oligonucléotides bifonctionnels, l'un se liant spécifiquement au nucléotide de type sauvage au niveau de la VNP de la seconde séquence cible, et l'autre se liant spécifiquement au nucléotide variant au niveau de la VNP de la seconde séquence cible, éventuellement dans lequel les parties spécifiques à l'ancrage des au moins deux oligonucléotides bifonctionnels se lient spécifiquement à différents ancrages immobilisés sur la surface.
